# EUROPEAN PATENT APPLICATION

(11) **EP 1 801 240 A1**
(43) Date of publication of application: **27.06.2007**
(21) Application number: 07075049.2
(22) Date of filing: 05.06.2002
(51) Int. Cl.: C12Q 1/68

(54) **Methods and compositions for assessment of pulmonary function and disorders**

(30) Priority: 05.06.2001 NZ 51216901; 17.07.2001 NZ 51301601; 18.09.2001 NZ 51427501
(62) Divisional of application: 02756005.1
(71) Applicant: AUCKLAND UNISERVICES LIMITED, Auckland (NZ)
(72) Inventor: Young, Robert Peter, Pamell, Auckland (NZ)
(74) Representative: Lee, Nicholas John

(57) **Abstract**

The present invention is concerned with methods for the assessment of pulmonary function and/or disorders, and in particular for diagnosing predisposition to and/or severity of chronic obstructive pulmonary disease in smokers and non-smokers using analysis of genetic polymorphisms and altered gene expression, particularly with regard to genes involved in matrix remodelling, anti-oxidant defence and the inflammatory response.

### Annex to the application documents - subsequently filed sequences listing

SEQUENCE LISTING

<110>  AUCKLAND UNISERVICES LIMITED

<120>  METHODS AND COMPOSITIONS FOR ASSESSMENT OF PULMONARY FUNCTION AND
        DISORDERS

<130>  P36299EP1

<140>  EP 07075049.2
<141>  2002-06-05

<150>  EP 02756005.1
<151>  2002-06-05

<150>  NZ 512169
<151>  2001-06-05

<150>  NZ 513016
<151>  2001-07-17

<150>  NZ 514275
<151>  2001-09-18

<150>  PCT/NZ02/00106
<151>  2002-06-05

<160>  15

<170>  PatentIn version 3.2

<210>  1
<211>  20
<212>  DNA
<213>  Artificial Sequence

<220>
<223>  PRIMER

<400>  1
gcctggcaca tagtaggccc                                                    20


<210>  2
<211>  20
<212>  DNA
<213>  Artificial Sequence

<220>
<223>  PRIMER

<400>  2
cttcctagcc agccggcatc                                                    20


<210>  3
<211>  25
<212>  DNA
<213>  Artificial Sequence

<220>

<223>  PRIMER

<400>  3
agatagtcaa gggatgatat cagct                                          25


<210>  4
<211>  21
<212>  DNA
<213>  Artificial Sequence

<220>
<223>  PRIMER

<400>  4
ggcttgtaga gctgttcagg g                                              21


<210>  5
<211>  21
<212>  DNA
<213>  Artificial Sequence

<220>
<223>  PRIMER

<400>  5
ctaccaggaa tggccttgtc c                                              21


<210>  6
<211>  21
<212>  DNA
<213>  Artificial Sequence

<220>
<223>  PRIMER

<400>  6
ctctcaggtc tgtgttcatc c                                              21


<210>  7
<211>  20
<212>  DNA
<213>  Artificial Sequence

<220>
<223>  PRIMER

<400>  7
ctgccctact tgattgatgg                                                20


<210>  8
<211>  20
<212>  DNA
<213>  Artificial Sequence

<220>
<223>  PRIMER

<400>  8
atcttctcct cttctgtctc                                                    20


<210>  9
<211>  20
<212>  DNA
<213>  Artificial Sequence

<220>
<223>  PRIMER

<400>  9
ttctggattg tagcagatca                                                    20


<210>  10
<211>  20
<212>  DNA
<213>  Artificial Sequence

<220>
<223>  PRIMER

<400>  10
accacaccag aaatgttcgc                                                    20


<210>  11
<211>  24
<212>  DNA
<213>  Artificial Sequence

<220>
<223>  PRIMER

<400>  11
agtagccaca gcggtagcag ctgc                                               24


<210>  12
<211>  28
<212>  DNA
<213>  Artificial Sequence

<220>
<223>  PRIMER

<400>  12
gcaaccaggc cagcgtggag aacgggaa                                           28


<210>  13
<211>  24
<212>  DNA
<213>  Artificial Sequence

<220>
<223>  PRIMER

<400>  13
ccagaggaga agctcaaagg caga                                              24


<210>  14
<211>  23
<212>  DNA
<213>  Artificial Sequence

<220>
<223>  PRIMER

<400>  14
caaagcagaa tcaagatgtc aat                                               23


<210>  15
<211>  21
<212>  DNA
<213>  Artificial Sequence

<220>
<223>  PRIMER

<400>  15
ctgggttaag caacacaaag c                                                 21

## Description

### TECHNICAL FIELD

The present invention is concerned with methods for assessment of pulmonary function and/or disorders, and in particular for diagnosing predisposition to and/or severity of chronic obstructive pulmonary disease in smokers and non-smokers using analysis of genetic polymorphisms and altered gene expression. The present invention is also concerned with methods for diagnosing impaired lung function and in particular to diagnosing predisposition to and/or severity of impaired lung function and the associated morbidity/mortality risk of other diseases. The invention also relates to compositions for use in said methods.

### BACKGROUND ART

Chronic obstructive pulmonary disease (COPD) is the 4^{th} leading cause of death in developed countries and a major cause for hospital readmission world-wide. It is characterised by insidious inflammation and progressive lung destruction. It becomes clinically evident after exertional breathlessness is noted by affected smokers when 50% or more of lung function has already been irreversibly lost. This loss of lung function is detected clinically by reduced expiratory flow rates (specifically forced expiratory volume in one second or FEV1). Over 95% of COPD is attributed to cigarette smoking yet only 20% or so of smokers develop COPD (susceptible smoker). Studies surprisingly show that smoking dose accounts for only about 16% of the impaired lung function. A number of family studies comparing concordance in siblings (twins and non-twin) consistently show a strong familial tendency and the search for COPD disease-susceptibility (or disease modifying) genes is underway.

Despite advances in the treatment of airways disease, current therapies do not significantly alter the natural history of COPD with progressive loss of lung function causing respiratory failure and death. Although cessation of smoking has been shown to reduce this decline in lung function if this is not achieved within the first 20 years or so of smoking for susceptible smokers, the loss is considerable and symptoms of worsening breathlessness can not be averted. Smoking cessation studies indicate that techniques to help smokers quit have limited success. Analogous to the discovery of serum cholesterol and its link to coronary artery disease, there is a need to better understnd the factors that contribute to COPD so that tests that identify at risk smokers can be developed and that new treatments can be discovered to reduce the adverse effects of smoking.

A number of epidemiology studies have consistently shown that at exposure doses of 20 or more pack years, the distribution in lung function tends toward trimodality with a proportion of smokers maintaining normal lung function (resistant smokers) even after 60+ pack years, a proportion showing modest reductions in lung function who may never develop symptoms and a proportion who show an accelerated loss in lung function who invariably develop COPD. This suggests that amongst smokers 3 populations exist, those resistant to developing COPD, those at modest risk and those at higher risk (termed susceptible smokers).

The underlying pathophysiology of COPD is not yet understood. Exposure to cigarette smoke in the lung results in both an inflammatory response and oxidant burden that initiates at least four processes. A number of cytokines are released locally and both neutrophils and macrophages are recruited in to the lung.

COPD is a heterogeneous disease encompassing, to varying degrees, emphysema and chronic bronchitis which develop as part of a remodelling process following the inflammatory insult from chronic tobacco smoke exposure and other air pollutants. It is likely that many genes are involved in the development of COPD.

Further, epidemiological studies have shown that impaired lung function, measured by spirometric methods, provides a direct method of diagnosing the presence of and/or tendency toward obstructive lung disorders (eg chronic obstructive lung disease, asthma, brochiectasis and bronchiolitis) (Subramanian D, et al. 1994)) and an indirect method of assessing morbidity/mortality risk from other diseases such as coronary artery disease, stroke and lung cancer (Hole DJ, et al. 1996, Knuiman MW, et al. 1999, Sorlie PD, et al. 1989, Bang KM, et al. 1993, Rodriguez BL, et al. 1994 and Weiss ST, et al. 1995).

It has long been recognised that impaired lung function as currently measured by spirometric methods is the clinical basis for assessing the presence and severity of obstructive lung diseases such as chronic obstructive lung disease, asthma, bronchiectasis and bronchiolitis. In the presence of chronic smoking these disorders may be characterised by minimally reversible airways obstruction as reflected by low forced expiratory volume in one second (FEV1), low percent predicted forced expiratory volume in one second and reduced ratio of the forced expiratory volume in one second over the forced vital capacity (general reference).

Over the last 20 years epidemiological studies have examined the relationship between impaired lung function (most often FEV1) with mortality risk from non-COPD causes of death notably coronary artery disease (CAD), stroke and lung cancer. Although these are well recognised consequences of chronic cigarette smoke exposure, it is clear from epidemiological studies that only a proportion of smokers die from these complications. However the converse shows that over 90% of COPD and lung cancers are attributable to chronic cigarette smoke exposure. Smoking is considered the most important lifestyle risk factor that contributes to coronary artery disease mortality. Although the epidemiological studies have recruited a diverse group of subjects they consistently identify reduced FEV1 (or related measure) as a significant and independent risk factor for death from not only COPD but also from CAD, stroke and lung cancer (Hole DJ, et al. 1996, Knuiman MW, et al. 1999, Sorlie PD, et al. 1989, Athonisen NR. 1989, Bang KM, et al. 1993, Rodriguez BL, et al. 1994 and Weiss ST, et al. 1995). In the largest of these studies, the risk of death from CAD attributed to impaired lung function was as great as that for serum cholesterol (Hole DJ, et al. 1996). The mortality risk associated with reduced FEV1 for CAD is seen for both smokers and non-smokers but is approximately two fold stronger in the former (Hole DJ, et al. 1996).

It has long been recognised that chronic obstructive lung disease, lung cancer, stroke and coronary artery disease all run in families and that chronic cigarette smoke exposure is an important environmental contributor to the development of these diseases. Although the prospective studies have shown that reduced FEV 1 is a reliable predictor of increased risk to all these diseases, it can take thirty or more years of chronic smoking before there is sufficient damage to the lungs for this susceptibility to be clinically detectable (by reduced lung function testing).

It would be desirable and advantageous to have methods which could be used to assess a subject's predisposition to developing pulmonary disorders such as chronic obstructive pulmonary disease (COPD), or a predisposition to developing impaired lung function and the associated risk of obstructive lung disease and risk for related diseases such as CAD, stroke and lung cancer, particularly if the subject is a smoker and/or has enhanced susceptibility to oxidative stress.

### SUMMARY OF THE INVENTION

The present invention is based in part on the surprising finding that promoter polymorphisms of certain genes and groups of genes, particularly those involved in matrix remodelling, anti-oxidant defence, inflammatory response and their inhibitors, are found more often in patients with COPD than in control subjects. In another part, the present invention is based on a further surprising finding that polymorphisms in these genes and/or their regulatory regions are associated with the severity of impaired lung function in smokers.

To date it has not been possible to assess the collective effects (additive, protective or multiplicative) of several "susceptibility" genetic variants in contributing to COPD/emphysema or impaired lung function. Thus, the presence of several variants (mutations and/or polymorphisms) of the COPD/emphysema susceptibility genes appears to confer a greater risk of having COPD/emphysema and/or impaired lung function in a simple additive model.

Thus, according to one aspect there is provided a method of determining a subject's predisposition to developing chronic obstructive pulmonary disease, comprising the analysis of polymorphisms in the regulatory and/or promoter regions of the genes encoding matrix metalloproteinase, wherein the presence of a mutation in these regions is indicative of predisposition to developing the disease.

According to another aspect there is provided a method of determining predisposition to developing chronic obstructive pulmonary disease in a subject who is a smoker or someone exposed to high levels of air pollutants such as environmental tobacco smoke, comprising the analysis of polymorphisms in the regulatory and/or promoter region of a matrix metalloproteinase, wherein the presence of a mutation in the regulatory and/or promoter regions of genes encoding matrix metalloproteinases is indicative of predisposition to developing the disease.

According to another aspect there is provided a method of diagnosing in a subject the potential onset of chronic obstructive pulmonary disease comprising the analysis of polymorphism in the regulatory and/or promoter regions of a matrix metalloproteinase, wherein the presence of a mutation in the promoter region of a matrix metalloproteinase is indicative of potential onset or severity of the disease.

According to another aspect there is provided a method of diagnosing in a subject who is a smoker or someone exposed to high levels of air pollutants such as environmental tobacco smoke the potential onset of chronic obstructive pulmonary disease comprising the analysis of polymorphism in the regulatory and/or promoter region of a matrix metalloproteinase, wherein the presence of a mutation in the promoter region of a matrix metalloproteinase is indicative of potential onset or severity of the disease.

Preferably the metalloproteinase is selected from interstitial collagenase (matrix metalloproteinase-1 or MMP-1), gelatinase B (matrix metalloproteinase-9 or MMP-9) and human macrophage elastase gene (MMP-12). The preferred polymorphism (mutation) is 1G/2G in the promoter of interstitial collagenase, C-1562T in the promoter of gelatinase B and the A to G -82 polymorphism in the promoter of human macrophage elastase gene and/or polymorphisms in linkage disequilibrium with these polymorphisms.

It will be understood by those skilled in the art that the determination of regulatory and/or promoter polymorphism may be conducted on one or a combination of the metalloproteinase genes. The assessment of predisposition of a subject to developing COPD may thus be based on the analysis of for example only the promoter region of interstitial collagenase or that of gelatinase B or that of macrophage elastase. It is preferred however that regulatory and/or promoter polymorphisms of a combination of these genes, two or all three in any combination, be used in the methods of the present invention.

According to yet another aspect there is provided a method of determining a subject's predisposition to developing chronic obstructive pulmonary disease, comprising the analysis of a combination of polymorphisms in the regulatory and/or promoter regions of the genes involved in, or associated with, matrix remodelling, wherein the presence of a polymorphism in these regions is indicative of predisposition to developing the disease.

Preferably, polymorphism in all three matrix metalloproteinase genes, namely interstitial collagenase (matrix metalloproteinase-1 or MMP-1), gelatinase B (matrix metalloproteinase-9 or MMP-9) and human macrophage elastase gene (MMP-12), is used in the methods of the present invention.

However, in conjunction with the polymorphisms in the metalloproteinase genes, polymorphisms in other known genes, such as alphal-antitrypsin and glutathione S-transferase, can be used in the methods of the present invention. Any combination of two or more gene polymorphisms may be used in the methods of the present invention however, for preference, the polymorphisms in all genes described herein is used.

According to another aspect there is provided a set of nucleotide probes and/or primers for use in the methods of any one of the previous aspects.

The preferred primers and/or probes are those which span, or are able to be used to span, the polymorphic regions of promoters and regulatory regions of the relevant genes described herein.

It will be understood that in the context of the present invention the term "polymorphisms" is used to describe any variants and mutations, including the total or partial absence of genes (eg. null mutations).

Method of determining a subject's potential risk of developing chronic obstructive pulmonary disease,comprising the analysis of polymorphisms in the regulatory and/or promoter regions of the genes encoding matrix metalloproteinases, inflammatory and anti-inflammatory cytokines, inhibitors of matrix metalloproteinases and enzymes involved in metabolising oxidants, wherein the presence of a mutation in these regions is indicative of a genotype protective against COPD.

Method of determining the potential risk of developing chronic obstructive pulmonary disease in a subject who is a smoker or someone exposed to high levels of air pollutants such as environmental tobacco smoke, comprising the analysis of polymorphisms in the regulatory and/or promoter regions of the genes encoding matrix metalloproteinases, inflammatory and anti-inflammatory cytokines, inhibitors of matrix metalloproteinases and enzymes involved in metabolising oxidants, wherein the presence of a mutation in these regions is indicative of a genotype protective against COPD.
Preferably, the metalloproteinase is interstitial collagenase (matrix metalloproteinase-1 or MMP-1). The preferred polymorphism (mutation) is 1G/2G in the promoter of interstitial collagenase. The preferred inflammatory cytokine is transforming growth factor bl, and the preferred polymorphism (mutation) is substitution of nucleotide T to C in codon 10 of transforming growth factor b1. The preferred inhibitor of matrix metalloproteinases is Tissue Inhibitor of Metalloproteinases 3 , and the preferred polymorphism is substitution of T to C at position -1296. The preferred enzyme involved in metabolising oxidants is superoxide dismutase 3 and the preferred polymorphism (mutation) is a substitution of C to G at position 760.

In yet another aspect there is provided a method of determining a subject's potential risk of developing chronic obstructive pulmonary disease, comprising the analysis of polymorphisms in the regulatory and/or promoter regions of the genes encoding matrix metalloproteinases, glutathione transferases, and protease inhibitors, wherein the presence of a mutation in these regions is indicative of a genotype susceptible to COPD.

In a further aspect there is provided a method of determining the potential risk of developing chronic obstructive pulmonary disease in a subject who is a smoker or someone exposed to high levels of air pollutants such as environmental tobacco smoke, comprising the analysis of polymorphisms in the regulatory and/or promoter regions of the genes encoding matrix metalloproteinases, glutathione transferases, and protease inhibitors, wherein the presence of a mutation in these regions is indicative of a genotype susceptible to COPD.
Preferably, the metalloproteinase is macrophage elastase (matrix metalloproteinase-12 or MMP-12) and the preferred polymorphism is substitution of A to G at position -82. The preferred glutathione transferase is glutathione S transferase 1 and the preferred polymorphism is the M1 null polymorphism. The preferred protease inhibitor is alpha 1 antitrypsin and the preferred polymorphism is the 3' Taq 1 polymorphism.

According to another aspect there is provided a method of determining a subject's predisposition to developing impaired lung function, comprising the analysis of polymorphisms in genes encoding matrix remodelling proteins (including proteases and/or their inhibitors), inflammatory proteins and oxidative stress responsive proteins, wherein the presence of a mutation in one or more of said genes is indicative of predisposition to developing impaired lung function and its associated health risks.

According to another aspect there is provided a method of determining predisposition to developing impaired lung function in a subject who is a smoker or a subject exposed to high levels of oxidative stress, comprising the analysis of polymorphisms in the genes encoding matrix remodelling proteins (including proteases and/or their inhibitors), inflammatory proteins and oxidative stress responsive proteins, wherein the presence of a mutation in one or more of the genes encoding the said proteins is indicative of predisposition to developing impaired lung function.

According to another aspect there is provided a method of diagnosing in a subject the potential onset of impaired lung function comprising the analysis of polymorphism in the genes encoding matrix remodelling proteins (including proteases and/or their inhibitors), inflammatory proteins and oxidative stress responsive proteins, wherein the presence of a mutation in one or more of the genes encoding matrix remodelling proteins (including proteases and/or their inhibitors), inflammatory proteins and oxidative stress responsive proteins is indicative of potential onset or severity of lung function impairment.

According to another aspect there is provided a method of diagnosing in a subject who is a smoker or a subject exposed to oxidative stress the potential onset of impaired lung function comprising the analysis of polymorphism in genes encoding matrix remodelling proteins (including proteases and/or their inhibitors), inflammatory proteins and oxidative stress responsive proteins, wherein the presence of a mutation in said proteins is indicative of potential onset or severity of lung function impairment.

In yet another aspect there is provided a method of determining a subject's predisposition to developing morbidity/mortality risk of a disease associated with impaired lung comprising the analysis of polymorphisms in the genes encoding proteins involved in matrix remodelling, inflammation and response to oxidative stress, wherein the presence of mutations in one or more of said genes is indicative of predisposition to developing morbidity/mortality risk of said disease.

Preferably the disease is selected from a group consisting of chronic obstructive lung diseases, coronary artery disease, stroke and lung cancer.

Preferably the matrix remodelling protein is selected from alphal-antitrypsin, interstitial collagenase (matrix metalloproteinase-1 or MMP-1), gelatinase B (matrix metalloproteinase-9 or MMP-9) and human macrophage elastase gene (MMP-12). Preferably, the oxidative stress protein is glutathione -s transferase. The preferred polymorphism (mutation) is the G to A 1237 in the 3' region of alphal -antitrypsin, the 1G/2G in the promoter of interstitial collagenase, C-1562T in the promoter of gelatinase B and the A to G -82 polymorphism in the promoter of human macrophage elastase gene and/or polymorphisms in linkage disequilibrium with these polymorphisms. Preferably the oxidative stress protein is selected from glutathione -s transferases.

It will be understood by those skilled in the art that the determination of susceptibility polymorphisms may be conducted on one or a combination of the genes encoding matrix remodelling proteins (including proteases and/or their inhibitors), inflammatory proteins and oxidative stress responsive proteins. The assessment of predisposition of a subject to developing impaired lung function may thus be based on the analysis of for example only the promoter region of interstitial collagenase or that of gelatinase B or that of macrophage elastase. It is preferred however that polymorphisms of a combination of these genes, two or all three in any combination, be used in the methods of the present invention.

According to yet another aspect there is provided a method of determining a subject's predisposition to developing impaired lung function, comprising the analysis of more than one polymorphism in the genes encoding matrix remodelling proteins (including proteases and/or their inhibitors), inflammatory proteins and oxidative stress responsive proteins, wherein the presence of a mutation in said genes is indicative of predisposition to developing impaired lung function.

Preferably, polymorphism in all five genes, namely alphal-antitrypsin, interstitial collagenase (matrix metalloproteinase-1 or MMP-1), gelatinase B (matrix metalloproteinase-9 or MMP-9), human macrophage elastase gene (MMP-12) and glutathione s-transferase, is used in the methods of the present invention.

According to another aspect there is provided a set of nucleotide probes and/or primers for use in the methods of any one of the previous aspects.

The preferred primers and/or probes are those which span, or are able to be used to span, the polymorphic regions of genes encoding matrix remodelling proteins (including proteases and/or their inhibitors), inflammatory proteins and oxidative stress responsive proteins and of other genes used in the methods of the present invention.

When referring to "smokers" herein the term is also intended to encompass ex-smokers.

### BRIEF DESCRIPTION OF FIGURES

- **Figure 1:**: The percentage of people with COPD plotted against the number of susceptibility genetic variants show a linear relationship with an estimated likelihood of having COPD as high as 80% in those with four or more susceptibility variants.
- **Figure 2:**: Graphic representation of the data in Table 10. The risk estimate, as quantified by the standardised ratio, shows that the presence of 0 protective genotypes in this study significantly increases the risk of a smoker having COPD by 167%. Conversely, this analysis shows that the risk of a smoker, with 2 or more protective genotypes, having COPD is reduced by 67%. Given the background risk of COPD in smokers before genetic testing is about 20%, the presence of 0 protective genotypes significantly increases this risk.
- **Figure 3:**: Graphical representation of the data in Table 11. Risk estimate analyses showed no significant differences in risk although trends showing a greater risk of a smoker having COPD in the presence of 2 or more susceptibility genotypes was evident.
- **Figure 4-6:**: Figures 4-6 show a trend towards greater lung function in smokers with increasing numbers of protective genotypes.

### DESCRIPTION OF THE PREFERRED EMBODIMENTS

Using case-control studies we have compared the frequencies of several genetic variants (polymorphisms) of candidate genes in smokers and blood donors. The majority of these candidate genes have confirmed (or likely) functional effects on gene expression or protein function. Specifically we have compared the frequencies of polymorphisms between blood donor controls, resistant smokers and those with COPD (subdivided into those with early onset and those with normal onset). The present invention demonstrates that there are both protective and susceptibility genotypes derived from selected candidate gene polymorphisms. Given COPD is a polygenic disorder and many genotypes are likely to be involved in the development of COPD in any one susceptible smoker only simple genetic models can be explored (ie the net effect of susceptibility and protective genotypes is not known). In one embodiment described herein 3 susceptibility genetic polymorphisms and 4 protective genetic polymorphisms are identified. Statistical analyses of the combined effects of these polymorphisms shows that the genetic assays of the present inventon can be used to identify smokers at greater risk of developing COPD.

Thus, through systematic analysis of the frequency of these polymorphisms in well defined groups of smokers and non-smokers, as described herein, it is possible to implicate certain proteins in the development of COPD and improve our ability to identify which smokers were at increased risk of developing impaired lung function and COPD for predictive purposes.

As only the minority of smokers suffer one or combination of these diseases, there may exists "susceptible smokers" who through the combined effects of genetic mechanisms and high oxidant exposure are at greatest risk of smoking related lung disease (COPD and lung cancer). Given the epidemiological findings to date it is likely that this genetic susceptibility also extends to a predisposition to other smoking related disorders such as CAD and stroke.

Further, we believe that reduced FEV 1 is a biomarker of a general susceptibility to the adverse effects of chronic smoking and oxidative stress. That is, under conditions of chronic oxidative stress (as in that seen with chronic cigarette smoke exposure) there is an alteration in the activity of proteins involved in matrix remodelling, inflammation and/or oxidative stress. The altered activities of these proteins (typically enzymes) over prolonged periods leads to the promotion of lung inflammation, fibrosis and parenchymal damage causing chronic obstructive lung diseases and contributes to lung cancer. These same processes occur in the arterial wall thereby promoting progression and/or instability of the atheromatous plaque resulting in, if not the development of coronary artery disease and stroke, the progression of atheromatous plaque to thrombus formation (Waltenberger J. 2001). Thrombus formation in turn leads to the clinical entities of unstable angina, acute myocardial infarction and stroke, all of which carry high mortality. It has been proposed that the processes of inflammation, matrix remodelling and/or response to oxidative stress may contribute to the plaque instability that characterises these clinical entities. In this context, reduced FEV 1 is an indirect biomarker of those with a susceptibility to adverse matrix remodelling, inflammation and enhanced damage from oxidative stress. Thus, through these mechanisms reduced FEV 1 and mortality risk for these diseases can be linked through the activity of matrix remodelling proteins, inflammatory proteins and an individual's inherent response to oxidative stress. Although reduced FEV1 is a reliable predictor of increased risk to all these diseases, it can take thirty or more years of chronic smoking before there is sufficient damage to the lungs for this susceptibility to be clinically detectable (by reduced lung function testing). The methods of the present invention overcome this disadvantage.

### EXAMPLES

The invention will now be described in more detail, with reference to nonlimiting examples.

### Example 1. Preliminary Study

### 1.1 Subject recruitment

Patients of European descent admitted to hospital with an exacerbation of their COPD were recruited consecutively. COPD was defined in subjects who had smoked a minimum of twenty pack years and had an FEV1/FVC¹ ratio (Forced expiratory volume in one second/Forced vital capacity) of < 70% and FEV1 as a percentage of predicted <70% (measured using American Thoracic Society criteria). Patients with the above lung function tests who had been diagnosed with COPD by specialist physicians were recruited if they were over 50 years old and had developed symptoms of breathlessness after 40 years of age. Those with a history of asthma, bronchiectasis or lung surgery were excluded. Eighty-four patients were recruited, of these 56% were male, the mean FEV1/FVC (± Standard Deviation) was 0.44 (0.12), mean FEV1 as a percentage of predicted was 33 (13). Mean age and pack year history was 73 yrs (9) and 45 pack years (29) respectively. Using a PCR based method (Sandford et al., 1999), we genotyped the COPD group for the α1-antitrypsin mutations (S and Z variants) and none had the Z allele (MZ, SZ, ZZ genotype). We also studied 178 European blood donors (smoking status unknown) who were recruited consecutively through the local blood donor service. Fifty-five percent were men and their mean age was 45 years.

### 1.2. MMP1 promoter polymorphism genotyping

Genomic DNA was extracted from whole blood samples (Maniatis,T., Fritsch, E. F. and Sambrook, J., Molecular Cloning Manual. 1989). The MMP1 promoter polymorphism was determined by minor modifications of a previously published method (Dunleavey et al., 2000, incorporated in its entirety herein by reference). The PCR oligonucleotide primers were 5'-TCG-TGA-GAA-TGT-CTT-CCC-ATT-3' (forward primer) and 5'-TCT-TGG-ATT-GAT-TTG-AGA-TAA-GTG-AAA-TC-3' (reverse primer). The PCR reaction was carried out in a total volume of 25ul and contained 50 ng genomic DNA, 10pmol forward and reverse primers, 100mM dNTPs, 10 mM Tris-HCL (pH 8.4), 50 mM KCl, 1.5 mM MgCl₂ and 1 unit of Taq polymerase. Cycling times were incubations for 2 min at 95°C followed by 35 cycles of 45s at 92°C, 50s at 60°C and 50s at 72°C. 4ul of PCR products (118bp) were visualised by ultraviolet trans-illumination of a 3% agarose gel stained with ethidium bromide. The remainder was digested for 4 hrs with 10 units of Asp 700 (Roche Diagnostics, New Zealand) at 37°C. Digested products were separated on a 3% agarose gel run for 2.5 hrs at 80 V with TBE buffer. The PCR product remained uncut (ie 118 bp) in the presence of the 2G allele or was cut in to bands of 100 bp and 17 bp in the presence of the 1 G allele Direct sequencing was performed in three subjects assigned the genotypes 1G1G, 1G2G or 2G2G by the above method and confirmed that the latter correctly identified the absence or presence of the 1 G or 2G alleles.

### 1.3. MMP 9 promoter polymorphism genotyping

Genomic DNA was extracted from whole blood by the same methods described in example 3. Genotyping of the gelatinase B (MMP 9) C-1562T promoter polymorphism was performed by PCR (modified from methods published by Zhang B, et al 1999, incorporated in its entirety herein by reference) using the primers gelb1 (5'-GCC-TGG-CAC-ATA-GTA-GGC-CC-3') and gelb2 (5-CTT-CCT-AGC-CAG-CCG-GCA-TC-3'). PCR amplification was performed in a PTC-100 thermo cycler (MJ Research, Inc.) in 25 µl reaction mix. The reaction mix was 50 ng of genomic DNA. 50 ng of each primer, 20µM of dNTP, 1.5mM MgCI2, 0.5 unit Taq DNA polymerase, 10mM Tris-HCl, 50mM KCI and 0.001%gelatin. The PCR cycle conditions were: An initial denaturation step at 95°C for 3 minutes, 35 cycles of PCR (denaturation at 92°C for 50 seconds, annealing at 66°C for 48 seconds, and elongation at 72°C for 58 seconds) followed by one cycle of elongation at 72°C for 5 minutes. Four microlitre aliquots of the PCR products were digested with 10 U restriction enzyme SphI (LifeTech) in the recommended buffer system at 37°C for five hours. All digests were analysed on a 3% agarose gel. Direct sequencing was performed in three subjects assigned the genotypes CC, CT or TT by the above method and confirmed that the latter correctly identified the C and T alleles.

### 1.4. MMP 12 promoter polymorphism genotyping

Genomic DNA was extracted from whole blood by the same methods described in example 3. Genotyping of the human macrophage elastase (MMP 12) A-82G promoter polymorphism was performed by PCR (modified from methods published by Jormsjo S et al 2000, incorporated in its entirety herein by reference) using the primers hmep1 (5'- AGA-TAG-TCA-AGG-GAT-GAT-ATC-AGC-T -3') and hmep2 (5- GGC-TTG-TAG-AGC-TGT-TCA-GGG -3'). PCR amplification was performed in a PTC-100 thermo cycler (MJ Research, Inc.) in 25 µl reaction mix. The reaction mix was 50 ng of genomic DNA. 50 ng of each primer, 20µM of dNTP, 1.5mM MgCI2, 0.5 unit Taq DNA polymerase, 10mM Tris-HCI, 50mM KCI and 0.001%gelatin. The PCR cycle conditions were: An initial denaturation step at 95°C for 2 minutes, 35 cycles of PCR (denaturation at 92°C for 50 seconds, annealing at 66°C for 48 seconds, and elongation at 72°C for 58 seconds) followed by one cycle of elongation at 72°C for 5 minutes. Four microlitre aliquots of the PCR products were digested with 10 U restriction enzyme PvuII (LifeTech) in the recommended buffer system at 37°C for five hours. All digests were analysed on a 3% agarose gel. Direct sequencing was performed in three subjects assigned the genotypes AA, AG or GG by the above method and confirmed that the latter correctly identified the A and G alleles

Genotypes were assigned by two investigators independently and blind to phenotype (COPD and control) status. The differences in allele and genotype frequencies were compared by odd's ratio using Cornfield 95% confidence limits, Yates corrected χ2-squared test with significance taken as p ≤ 0.05.

### 1.5. MMP1/MMP9/MMP12 promoter allele and genotype frequencies

Analysis of our genotyping data showed that Hardy Weinberg equilibrium was met in both cohorts and that significant differences were found (summarised in Tables 1, 2 and3).

**Table 1. MMP 1 1G/2G promoter polymorphism allele and genotype frequency in the COPD patients and blood donor controls.**

| Frequency | Allele* | | Genotype | | |
|---|---|---|---|---|---|
| | 1G | 2G | 1G1G | 1G2G | 2G2G |
| COPD n=84 (%) | 67 (40%) | 101¹ (60%) | 16 (19%) | 35 (42%) | 33² (39%) |
| Controls n=178 (%) | 197 (55%) | 159 (45%) | 56 (31%) | 85 (48%) | 37 (21%) |

| | | | | | |
|---|---|---|---|---|---|
| * number of chromosomes (2n) | | | | | |

1. Allele: 2G vs 1G for COPD vs controls, Odds ratio (OR) =1.89, 95% confidence limits 1. 3-2.7,
χ² (Yates corrected)=10.67, p=0.001
Genotype: 2G2G vs 1G1G + 1G2G for COPD vs controls, OR =2.47, 95% confidence limits 1.3-4.5, χ² (Yates corrected)=9.05, p=0.003

**Table 2. MMP 9 C-1562T promoter polymorphism allele and genotype frequency in the COPD patients and blood donor controls.**

| Frequency | Allele* | | Genotype | | |
|---|---|---|---|---|---|
| | C | T | CC | CT | TT |
| COPD n=84 (%) | 124 (74%) | 44¹ (26%) | 45 (53%) | 34 (40%) | 5² (7%) |
| Controls n=178 (%) | 301 (85%) | 55 (15%) | 126 (71%) | 49 (27%) | 3 (2%) |

| | | | | | |
|---|---|---|---|---|---|
| * number of chromosomes (2n)1. Allele: T vs C for COPD vs controls, Odds ratio | | | | | |

(OR) =1.94, 95% confidence limits 1.2-3. 1,
χ² (Yates corrected)=7.91, p=0.004
2. Genotype: CT/TT vs CC for COPD vs controls, OR =2.1,95% confidence limits 2.2-3.7,
χ² (Yates corrected)=6.7, p=0.009.

**Table 3. MMP 12 A-82G promoter polymorphism allele and genotype frequency in the COPD patients and blood donor controls.**

| Frequency | Allele* | | Genotype | | |
|---|---|---|---|---|---|
| | A | G | AA | AG | GG |
| COPD n=84 (%) | 151 (90%) | 17 (10%) | 67 (80%) | 17 (20%) | 0 (0%) |
| Controls n=178 (%) | 286 (80%) | 70 (20%) | 114 (64%) | 58 (33%) | 6 (3%) |

| | | | | | |
|---|---|---|---|---|---|
| * number of chromosomes (2n) | | | | | |

3. Allele: A vs G for COPD vs controls, Odds ratio (OR) =2.17, 95% confidence limits 1.8-2. 8,
χ² (Yates corrected)=6.83, p=0.009
2. Genotype: AA vs AG/GG for COPD vs controls, OR =2.21, 95% confidence limits 1.2-4.3,
χ² (Yates corrected)=5.89, p=0.02.

The above data indicate that the MMP1 1 G/2G, the MMP9 C-1562T and the MMP 12 A-82G promoter polymorphisms are independently candidate loci for susceptibility to the development of COPD. In case of MMP1, both the 2G allele frequency and the 2G2G genotype frequency were significantly more prevalent in the COPD patients than in the healthy control population (60% vs 45% and 39% vs 21 % respectively). Similarly, in case of MMP9 both the T allele frequency and the CT/TT genotype frequencies were significantly more prevalent in the COPD patients than in the healthy control population (26% vs 15% and 47% vs 29% respectively). Lastly, in case of MMP 12 both the A allele frequency and the AA genotype frequency were significantly more prevalent in the COPD patients than in the healthy control population (90% vs 80% and 80% vs 64% respectively).

### 1.6. Genotyping the alphal-antitrypsin 3' Taq 1 polymorphism.

Genomic DNA was extracted from whole blood as for the previous examples. The alphal-antitrypsin 3' Taq 1 polymorphism was determined by the following methods using previously published primers (Sandford AJ, et al 1997b). The PCR oligonucleotide primers were 5'-CTA-CCA-GGA-ATG-GCC-TTG-TCC-3' (forward primer) and 5'-CTC TCA GGT CTG TGT TCA TCC-3' (reverse primer). The PCR reaction was carried out in a total volume of 25ul and contained 50 ng genomic DNA, 10pmol forward and reverse primers, 100mM dNTPs, 10 mM Tris-HCL (pH 8.4), 50 mM KCl, 1.5 mM MgCl₂ and 1 unit of Taq polymerase. Cycling times were incubations for 2 min at 95°C followed by 35 cycles of 45s at 94°C, 40s at 62°C and 45s at 72°C with a final cycle of 5 mins at 72°C. 4ul of PCR products (205bp) were visualised by ultraviolet trans-illumination of a 1.5% agarose gel stained with ethidium bromide. The remainder of the PCr product was digested for 4 hrs with 10 units of Taq 1 restriction enzyme (Roche Diagnostics, New Zealand) at 65°C. Digested products were separated on a 2% agarose gel run for 2.5 hrs at 80 V with TBE buffer. The PCR product remained uncut (ie 205 bp) in the presence of the t (mutant) allele or was cut in to bands of 130 bp and 75 bp in the presence of the T (wild type) allele.

### 1.7. Genotyping the glutathione S-transferase (GST)M1 null polymorphism.

Genomic DNA was extracted from whole blood as for the previous examples. The glutathione S-transferase (GST)M1 null deletion polymorphism was determined by the following methods using previously published primers (Cantlay AM, et al. 1994). The PCR oligonucleotide primers were (5'-CTG-CCC-TAC-TTG-ATT-GAT-GG -3'; 5'-ATC-TTC-TCC-TCT-TCT-GTC-TC-3' and 5'-TTC-TGG-ATT-GTA-GCA-GAT-CA-3'). The PCR reaction was carried out in a total volume of 25ul and contained 50 ng genomic DNA, 50 ng of each primer, 20uM dNTPs, 10 mM Tris-HCL (pH 8.4), 50 mM KC1,1.5 mM MgCl₂ and 0.5 unit of Taq polymerase. Cycling times were incubations for 3 min at 95°C followed by 35 cycles of 45s at 94°C, 48s at 56°C and 48s at 72°C with a final elongation for 3 mins at 72°C. 6ul of PCR products were visualised by ultraviolet trans-illumination of a 2% agarose gel stained with ethidium bromide. The PCR products were a 202 bp band (GSTM4 internal control for amplification) and either a 275 bp band for a normal GSTM1 (homozygote or heterozygote) or no 275 bp band indicating homozygosity for the GSTM1 null deletion.

**Table 4. Alphl-antitrypsin 3' prime Taq 1 polymorphism allele and genotype frequency in the COPD patients and blood donor controls.**

| Frequency | Allele* | | Genotype | | |
|---|---|---|---|---|---|
| | T | t | TT | Tt | tt |
| COPD n=84 (%) | 150 (89%) | 18¹ (11%) | 66 (79%) | 18 (21 %) | 0 (0%) |
| Controls n=178 (%) | 345 (97%) | 11 (3%) | 167 (94%) | 11 (6%) | 0 (0%) |

| | | | | | |
|---|---|---|---|---|---|
| * number of chromosomes (2n) | | | | | |

4. Allele: t vs T for COPD vs controls, Odds ratio (OR) =3.76, 95% confidence limits 1.6-8. 8,
χ² (Yates corrected)=11.27, p=0.0008
2. Genotype: Tt /tt vs TT for COPD vs controls, OR =11.98, 95% confidence limits 1.7-10.0,
χ² (Yates corrected)=11.98, p=0.0005.

**Table 5. GSTM1 polymorphism allele and genotype frequency in the COPD patients and blood donor controls.**

| Frequency | | |
|---|---|---|
| | N | n |
| COPD n=84 (%) | 29 (35%) | 55 (65%) |
| Controls n=178 (%) | 103 (58%) | 75 (42%) |

n vs N in COPD compared to controls OR=2.6, 95% CI 1.5-4.6,
χ² (Yates corrected)=11.52, p=0.0007.

Although this polymorphism is located in a region containing regulatory elements, in vitro studies have failed to show an important functional effect from this genetic variant (Sandford et al. 1997). For the GSTM1 mutation, we found a frequency of 42% in controls and 65% in our COPD patients.

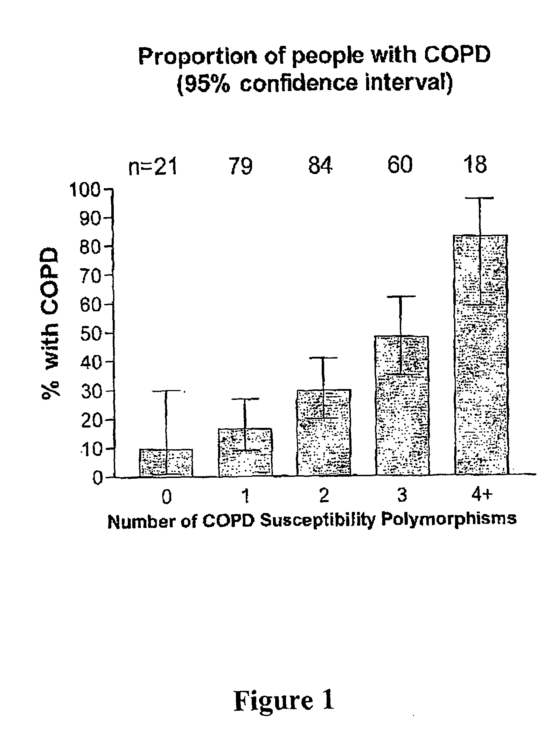

When the combined effect of these genetic variants were assessed in a logistic equation (Odds ratio estimates) which assumed similar frequencies in healthy smokers as our controls, it estimated that about 42% (95% CI=31-56%) of the tendency to COPD could be explained independently, by each of the five genetic variants. When examined graphically, the percentage of people with COPD plotted against the number of susceptibility genetic variants they had showed a linear relationship with an estimated likelihood of having COPD as high as 80% in those with four or more susceptibility variants (see figure 1).

Our COPD cohort is likely to be a heterogeneous group of patients with varying degrees of emphysema, bronchitis and reversible airways obstruction (the asthmatic component of COPD).

The present study provides evidence that MMP1, MMP 9 and/or MMP12 over-expression may underlie the development of COPD in some smokers and is the first to directly implicate the 2G allele (in MMP1), T allele (in MMP9) and G allele (in MMP12) promoter polymorphisms as the genetic basis of this process.

As stated above, the COPD cohort used in the present studies is likely to be a heterogeneous group of patients with varying severity of emphysema as part of their COPD. We propose that one and/or a combination of the MMP 1 2G allele and/or MMP9 T allele and/or MMP12 A allele, exert their effect on the development of COPD through over-expression of the relevant matrix metalloproteinase and subsequent development of emphysema. However, it is not clear how the inflammatory stimulus of chronic cigarette exposure results in MMP over-expression. In this regard it is of interest that tumour necrosis factor-α (TNF-α), an inflammatory cytokine released in response to cigarette smoke (and implicated in COPD remodeling), has been shown to bind to the core sequence of Ets transcription factor binding site (von der Ahe et al., 1993). While not wishing to be bound by any particular mechanism of action, it is noted that the above polymorphisms are associated (found near to or generate) Ets transcription binding sites which may enhance responsiveness to inflammatory stimuli such as TNF-α rendering those smokers at risk of developing emphysema.

### Example 2. Expanded Study.

The preliminary assessment of MMP1, 9 and 12 promoter polymorphisms as candidate loci for susceptibility to the development of COPD was followed by a second, broader study that included genotyping of candidate polymorphisms of the TGFβ, SOD3 and TIMP3 genes. This study was designed to investigate whether both protective and susceptibility genotypes may be derived from this subset of genes.

### 2.1 Subject recruitment

Subjects of European decent who had smoked a minimum of fifteen pack years and diagnosed by a physician with chronic obstructive pulmonary disease (COPD) were recruited from two sources. The first group were patients admitted with an exacerbation of their COPD and who met the following criteria: were over 50 years old and had developed symptoms of breathlessness after 40 years of age, had a Forced expiratory volume in one second (FEV1) as a percentage of predicted <70% and a FEV1/FVC² ratio (Forced expiratory volume in one second/Forced vital capacity) of< 70% (measured using American Thoracic Society criteria). One hundred and eleven subjects were recruited, of these 58% were male, the median FEV1/FVC (± interquartile range (IRQ)) was 44% (37-50), median FEV 1 as a percentage of predicted was 32 (24-47). Median age and pack year history was 73 yrs (66-77) and 43 pack years (30-57) respectively. The second group recruited were patients referred with breathlessness to a chest outpatient clinic who, after clinical and spirometric assessment, were diagnosed as having COPD (by a chest physician) and who met the following criteria: were less than 65 years old and had developed symptoms of breathlessness after 40 years of age, had a Forced expiratory volume in one second (FEV1) as a percentage of predicted <70% and a FEV1/FVC³ ratio (Forced expiratory volume in one second/Forced vital capacity) of < 70% (measured using American Thoracic Society criteria). Sixty-one subjects were recruited, of these 43% were male, the median FEV1/FVC (± interquartile range (IRQ)) was 44% (38-55), median FEV1 as a percentage of predicted was 37 (24-47). Median age and pack year history was 58 yrs (53-62) and 41 pack years (31-52) respectively. Using a PCR based method (Sandford et al., 1999), we genotyped both COPD groups for the α1-antitrypsin mutations (S and Z variants) and had excluded those with the Z allele (MZ, SZ, ZZ genotype). We also studied 85 European subjects who had smoked a minimum of twenty pack years and who had never suffered breathlessness and had not been diagnosed with an obstructive lung disease in the past, in particular asthma or chronic obstructive lung disease. This control group was recruited through clubs for the elderly and consisted of 58% male, the median FEV1/FVC (± IQR) was 82% (76-88), median FEV1 as a percentage of predicted was 94 (87-101). Median age and pack year history was 52 yrs (46-61) and 38 pack years (25-59) respectively. We also recruited 178 European blood donors (smoking status unknown) were recruited consecutively through local blood donor services. Fifty-five percent were men and their median age was 45 years.

This study shows that polymorphisms found in greater frequency in COPD patients compared to controls may reflect an increased susceptibility to the development of impaired lung function and COPD. Similarly, polymorphisms found in greater frequency in resistant smokers compared to susceptible smokers (COPD patients) may reflect a protective role.

### Summary of characteristics for the COPD (hosp), COPD (clinic) and resistant smokers.

| *Parameter* | *COPD, n=111* | *COPD, n=61* | *Resistant, n=85* |
|---|---|---|---|
| *Median (IQR)* | *Hosp. admission* | *Outpatient clinic* | *Healthy smokers* |
| *% male* | *58%* | *43%* | *58%* |
| *Age (yrs)* | *73 (66-77)* | *58(53-62)* | *52 (46-61)* |
| *Pack years* | *43 (30-57)* | *41(31-52)* | *38 (25-59)* |
| *FEV₁ (L)* | *0.76(0.55-1.6)* | *0. 93 (0.65-1.2)* | *2.8 (2.5-3.3)* |
| *FEV₁ % predicted* | *32% (24-47)* | *37% (24-47)* | *94% (87-101)* |
| *FEV₁*/*FVC* | *44 (37-50)* | *44 (38-55)* | *82 (76-86)* |
| | | | |

### 2.2. Transforming growth factor β (TGFβ) codon 10 polymorphism genotyping.

Genomic DNA was extracted from whole blood samples (Maniatis,T., Fritsch, E. F. and Sambrook, J., Molecular Cloning Manual. 1989). The (TGFβ) codon 10 polymorphism was determined by minor modifications of a previously published method (Syrris et al., 1998, incorporated in its entirety herein by reference)). The PCR oligonucleotide primers were 5'-ACC ACA CCA GAA ATG TTC GC-3' (forward primer) and 5'-AGT AGC CAC AGC GGT AGC AGC TGC - 3' (reverse primer). The PCR reaction was carried out in a total volume of 25ul and contained 20 ng genomic DNA, 500pmol forward and reverse primers, 0.2mM dNTPs, 10 mM Tris-HCL (pH 8.4), 150 mM KCl, 1.0 mM MgCl₂ and 1 unit of Taq polymerase (Life Technologies). Cycling times were incubations for 3 mins at 95°C followed by 33 cycles of 50s at 94°C, 60s at 66°C and 60s at 72°C. A final elongation of 10 mins at 72°C then followed. 4ul of PCR products were visualised by ultraviolet trans-illumination of a 3% agarose gel stained with ethidium bromide. An aliquot of 3ul of aplification product was digested for 1 hr with 4 units of PstI (Roche Diagnostics, New Zealand) at 37°C. Digested products were separated on a 2.5% agarose gel run for 2.0 hrs at 80 mV with TBE buffer. Using ultraviolet transillumination after ethidium bromide staining. The products were visualised against a 123bp ladder. In the presence of the leucine (L) allele the product size of 110 pb were cut in to 86bp and 24 bp products while the amplified product remained uncut in the presence of the proline (P) allele. Direct sequencing was performed in three subjects assigned the genotypes LL, LP and PP by the above method and confirmed that the latter correctly identified the absence or presence of the L or P alleles.

### 2.3. Superoxide dismutase 3 (SOD3) C+760G (Arg213Gly) polymorphism genotyping.

Genomic DNA was extracted from whole blood samples (Maniatis,T., Fritsch, E. F. and Sambrook, J., Molecular Cloning Manual. 1989). The SOD 3 C+760G polymorphism was determined by minor modifications of a previously published method (Ukkola et al., 2001, incorporated in its entirety herein by reference)). The PCR oligonucleotide primers were 5'-GCA ACC AGG CCA GCG TGG AGA ACG GGA A -3' (forward primer) and 5'-CCA GAG GAG AAG CTC AAA GGC AGA -3' (reverse primer). The PCR reaction was carried out in a total volume of 25ul and contained 175 ng genomic DNA, 1nmol forward and reverse primers, 0.1mM dNTPs, 10 mM Tris-HCL (pH 8.4), 150 mM KCl, 1.0 mM MgCl₂ and 0.5 unit of Taq polymerase (Life Technologies). Cycling times were incubations for 3 mins at 95°C followed by 33 cycles of 50s at 94°C, 60s at 66°C and 60s at 72°C. A final elongation of 10 mins at 72°C then followed. 4ul of PCR products were visualised by ultraviolet trans-illumination of a 3% agarose gel stained with ethidium bromide. An aliquot of 3ul of amplification product was digested for 1 hr with 10 units of MwoI (Roche Diagnostics, New Zealand) at 60°C. Digested products (221 bp)were separated on a 3.5% agarose gel run for 1.0 hrs at 80 V with TBE buffer. Using ultraviolet transillumination after ethidium bromide staining, the products were visualised against a 123bp ladder. In the presence of the Gly-213 (or G) allele the product size of 221 pb was uncut but in the presence of the wild-type Arg 213(or C) allele the product is cut in to 2 bands. Direct sequencing was performed in three subjects assigned the genotypes CC and CG by the above method and confirmed that the latter correctly identified the absence or presence of the C or G alleles.

### 2.4. Tissue inhibitor of metalloproteinase 3 (TIMP 3) T-1296C promoter polymorphism genotyping.

Genomic DNA was extracted from whole blood samples (Maniatis,T., Fritsch, E. F. and Sambrook, J., Molecular Cloning Manual. 1989). The TIMP 3 T-1296C promoter polymorphism was determined by minor modifications of a previously published method (Beranek., 2000, incorporated in its entirety herein by reference)). The PCR oligonucleotide primers were 5'-CAA AGC AGA ATC AAG ATG TCA AT -3' (forward primer) and 5'-CTG GGT TAA GCA ACA CAA AGC-3' (reverse primer). The PCR reaction was carried out in a total volume of 25ul and contained 1 ug genomic DNA, 10 pmol forward and reverse primers, 0.2mM dNTPs, 10 mM Tris-HCL (pH 8.4), 50 mM KCl, 1.5 mM MgCl₂ and 0.7 unit of Taq polymerase (Life Technologies). Cycling times were incubations for 5 mins at 95°C followed by 30 cycles of 30s at 95°C, 60s at 61°C and 30s at 72°C. A final elongation of 10 mins at 72°C then followed. 4ul of PCR products were visualised by ultraviolet trans-illumination of a 3% agarose gel stained with ethidium bromide. An aliquot of 10ul of amplification product was digested overnight with 5 units of AluI (Roche Diagnostics, New Zealand) at 37°C. Digested products (221 bp)were separated on a 3.5% agarose gel run for 1.0 hrs at 80 V with TBE buffer. Using ultraviolet transillumination after ethidium bromide staining, the products were visualised against a 123bp ladder. In the presence of the T allele the digest bands were 201, 128, 69, 53 and 32 while in the presence of the C allele the digest bands were 201, 160, 69, and 55 bands. Direct sequencing was performed in three subjects assigned the genotypes TT, TC and CC by the above method and confirmed that the latter correctly identified the absence or presence of the T or C alleles.

### Combined Results of the COPD genetic association study. (summarised table 9)

The comparisons shown in Tables 3,4 and 5 show that the MMP 12 A allele/AA genotype (-82 promoter), α1-antitrysin t allele/Tt/tt genotype (1237 3' region) and GSTM1 nn (null) genotype are significantly increased in frequency in COPD patients compared to controls. The comparisons shown in Tables 1, 6, 7 and 8 show that the MMP 1 2G allele/2G2G genotype (-1607 promoter), P allele/PP genotype (codon 10), G allele/CG genotype (+760 exonic) and TT genotype (-1296 promoter) are significantly increased in frequency in resistant smokers compared to COPD patients.

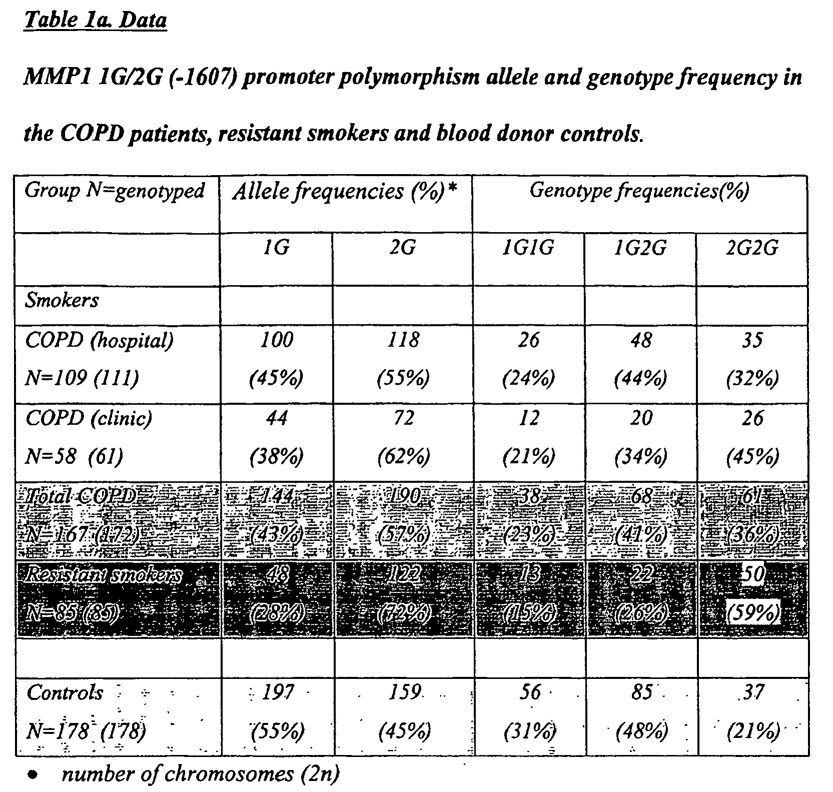

**Table 1b. Analysis**

| ***MMP1 1G*/*2G (-1607) promoter polymorphism 2x2 contingency table statistics comparing the allele and genotype frequencies in the COPD patients, resistant smokers and blood donor controls.*** | | | | | | | |
|---|---|---|---|---|---|---|---|
| *Groups* | *Allele*/*genotye* | *OR* | *95% CI for OR* | *χ2- M-H* | *P* | *χ2 Yates* | *P* |
| *2G vs 1G* | *Resist vs total COPD* | *1.93* | *1.27-2.93* | *10.53* | *0.001* | *9.95* | *0.001* |
| *2G2G vs 1G2G*/*1G1G* | *Resist vs total COPD* | *2.48* | *1.41-4.39* | *11.32* | *0. 0008* | *10. 48* | *0. 001* |
| | | | | | | | |

| | | | | | | | |
|---|---|---|---|---|---|---|---|
| *OR = odd's ratio, 95% CI = 95% confidence interval* *χ2 - M-H = χ2 Mantel-Haenszel, χ2 Yates = χ2 Yates corrected* | | | | | | | |

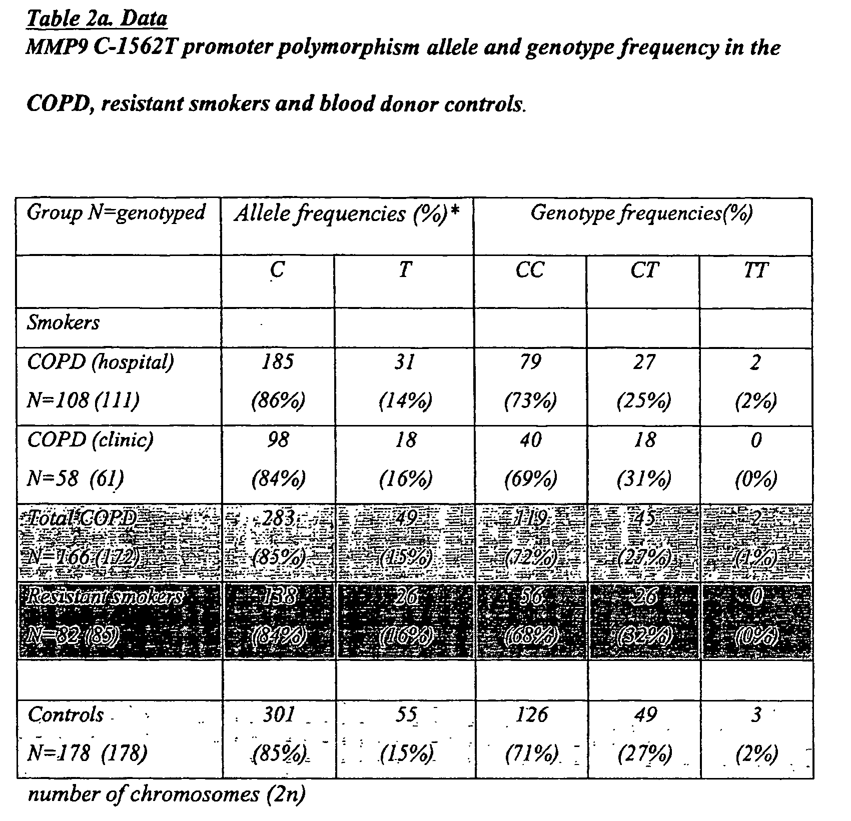

**Table 2b. Analysis**

| |
|---|
| ***MMP9 C-1562Tpromoterpolymorphism 2x2 contingency table statistics comparing the allele and genotype frequencies in the COPD patients, resistant smokers and blood donor controls.*** |
| *No significant differences observed* |

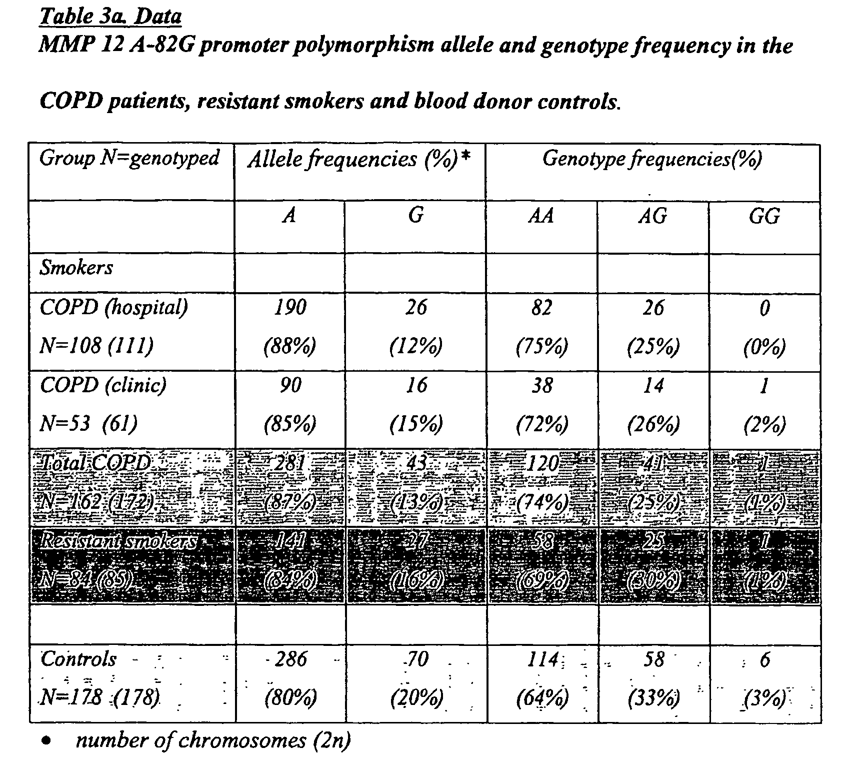

**Table 3b. Analysis**

| ***MMP 12 A-82G promoter polymorphism 2x2 contingency table statistics comparing the allele and genotype frequencies in the COPD patients, resistant smokers and blood donor controls.*** | | | | | | | |
|---|---|---|---|---|---|---|---|
| *Groups* | *Allele*/*genotye* | *OR* | *95% CI for OR* | *χ2-M-H* | *P* | *χ2 Yates* | *P* |
| *A vs G* | *Total* COPD *vs controls* | *1.60* | *1.04-2.47* | *4.99* | *0. 03* | *4.55* | *0. 03* |
| *AA vs AG* | *Total COPD vs controls* | *1.60* | *0.98-2.63* | *3.96* | *0.05* | *3.52* | *0. 06* |
| | | | | | | | |

| | | | | | | | |
|---|---|---|---|---|---|---|---|
| *OR = odd's ratio, 95% CI = 95% confidence interval* *χ2- M-H = χ2 Mantel-Haenszel, χ2 Yates = χ2 Yates corrected* | | | | | | | |

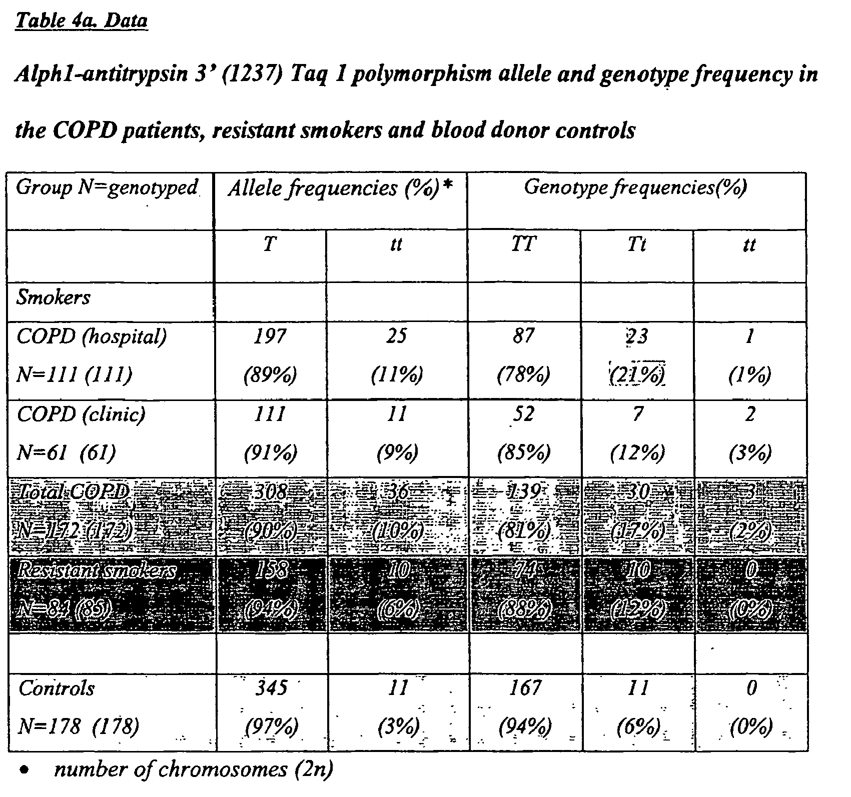

**Table 4b. Analysis**

| ***Alph1-antitrypsin 3' (1237) Taq 1 polymorphism 2x2 contingency table statistics comparing the allele and genotype frequencies in the COPD patients, resistant smokers and blood donor controls.*** | | | | | | | |
|---|---|---|---|---|---|---|---|
| *Groups* | *Allele*/*genotye* | *OR* | *95% CI for OR* | *χ2 - M-H* | *P* | *χ2 Yates* | *P* |
| *t vs T* | *COPD (hosp) vs controls* | *3. 98* | *1.83-8.82* | *15.63* | *0.00008* | *14.26* | *0.0002* |
| *Tt*/*tt vs TT* | *COPD (hosp) vs controls* | *4.19* | *1.86-9.60* | *15.26* | *0.00009* | *13.90* | *0.0002* |
| *t vs T* | *COPD total vs controls* | *3.67* | *1.76-7.79* | *15.17* | *0.0001* | *14.04* | *0.0002* |
| *Tt vs TT* | *COPD (hosp) vs controls* | *3.69* | *1.68-7.89* | *13.42* | *0.0002* | *12.31* | *0.0005* |
| *Tt vs TT* | *COPD (hosp) vs controls* | *3.69* | *1.68-7.89* | *13.42* | *0.0002* | *12.31* | *0.0005* |
| *t vs T* | *COPD (hosp) vs resistant* | *2.01* | *0.89-4.62* | *3.29* | *0.07* | *2.62* | *0.10* |
| *Tt*/*tt vs TT* | *COPD (hosp) vs resistant* | *2. 04* | *0.86-4.92* | *3.12* | *0.08* | *2.50* | *0.11* |

| | | | | | | | |
|---|---|---|---|---|---|---|---|
| *OR = odd's ratio, 95% CI = 95% confidence interval* *χ2 - M-H* = *χ2 Mantel-Haenszel, χ2 Yates = χ2 Yates corrected* | | | | | | | |

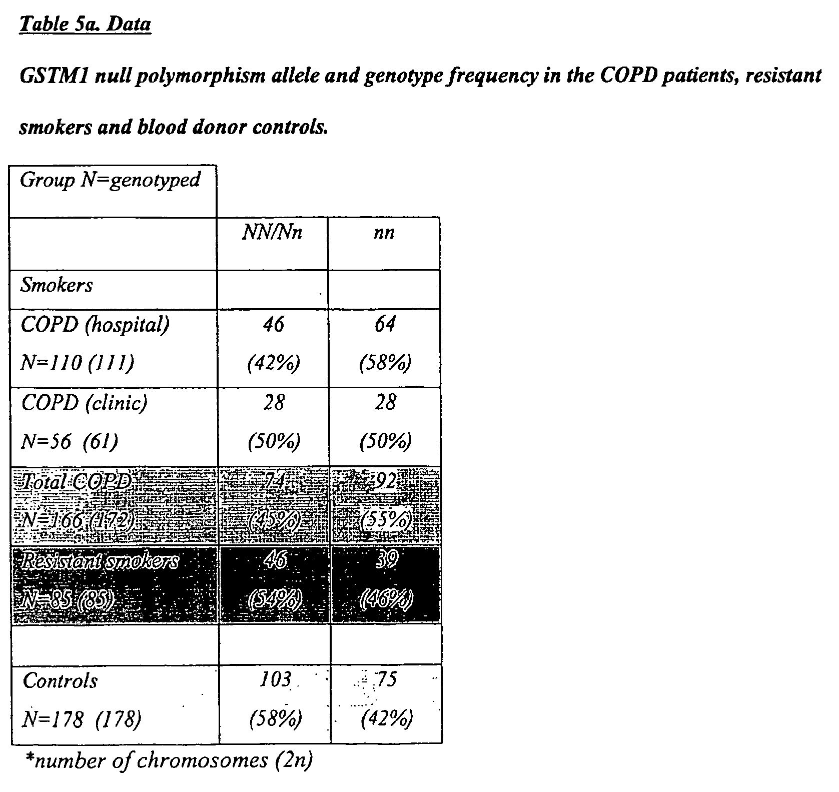

**Table 5b. Analysis**

| ***GSTM1 null polymorphism 2×2 contingency table statistics comparing the allele and genotype frequencies in the COPD patients, resistant smokers and blood donor controls.*** | | | | | | | |
|---|---|---|---|---|---|---|---|
| *Groups* | *Allele*/*genotye* | *OR* | *95% CI for OR* | *χ2*-*M-H* | *P* | *χ2 Yates* | *P* |
| *nn vs Nn*/*NN* | *Total COPD vs controls* | *1.71* | *1.09-2.68* | *6.05* | *0.01* | *5.55* | *0.02* |
| *nn vs Nn*/*NN* | *COPD (hosp) vs controls* | *1.91* | *1.15-3.19* | *6.99* | *0.008* | *6.38* | *0.01* |
| *nn vs Nn*/*NN* | *COPD (hosp) vs resistant* | *1.64* | *0.89-3.03* | *2.90* | *0.09* | *2.44* | *0.12* |

| | | | | | | | |
|---|---|---|---|---|---|---|---|
| *OR = odd's ratio, 95% CI = 95% confidence interval* *χ2- M-H = χ2 Mantel-Haenszel, χ2 Yates = χ2 Yates corrected* | | | | | | | |

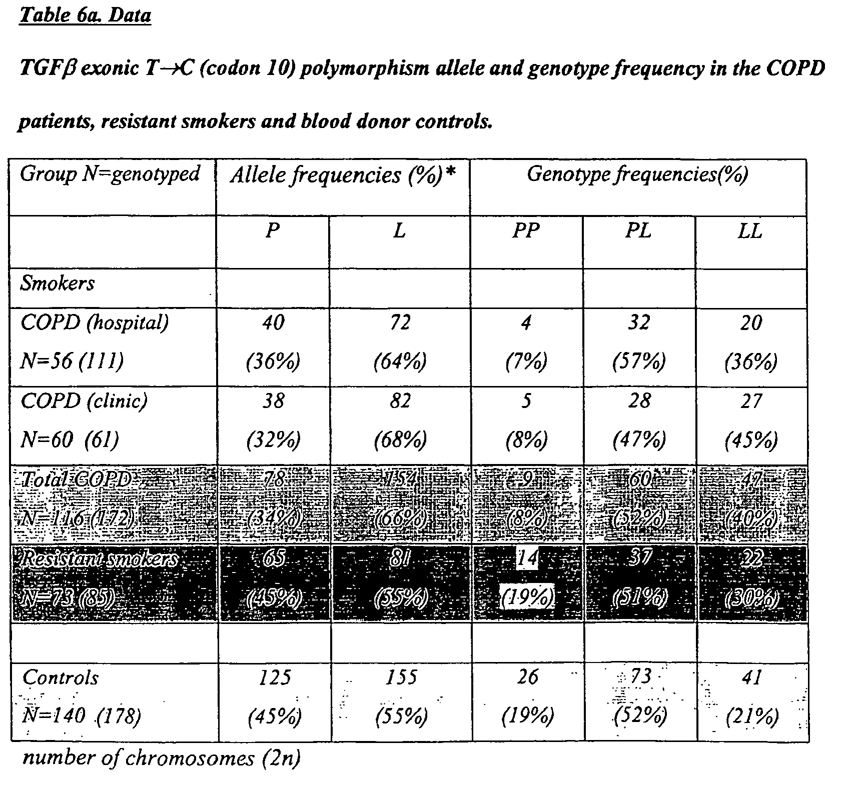

**Table 6b. Analysis**

| ***TGFβ exonic T→C (codon 10) polymorphism 2x2 contingency table statistics comparing the allele and genotype frequencies in the COPD patients, resistant smokers and blood donor controls.*** | | | | | | | |
|---|---|---|---|---|---|---|---|
| *Groups* | *Allele*/*genotye* | *OR* | *95% CI for OR* | *χ2 - M-H* | *P* | χ*2 Yates* | *P* |
| *P vs L* | *Resistant vs COPD (total)* | *1.58* | *1.01-2.48* | *4.51* | *0.03* | *4.07* | *0.04* |
| *PP vs PL*/*LL* | *Resistant vs COPD(total)* | *2.82* | *1.07-7.58* | *5.44* | *0.02* | *4.45* | *0. 03* |
| | | | | | | | |

| | | | | | | | |
|---|---|---|---|---|---|---|---|
| *OR = odd's ratio, 95% CI = 95% confidence interval* *χ2- M-H = χ2 Mantel-Haenszel, χ2 Yates = χ2 Yates corrected* | | | | | | | |

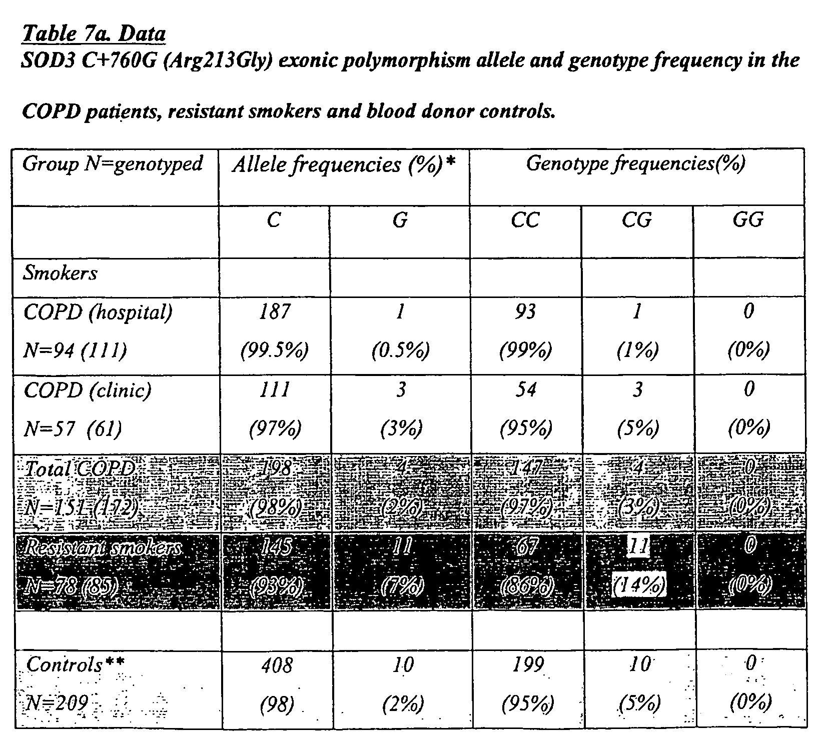

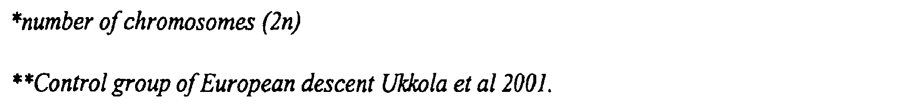

**Table 7b. Analysis**

| ***SOD3 C+760G (Arg213Gly) exonic polymorphism 2x2 contingency table statistics comparing the allele and genotype frequencies in the COPD patients, resistant smokers and blood donor controls.*** | | | | | | | |
|---|---|---|---|---|---|---|---|
| *Groups* | *Allele*/*genotye* | *OR* | *95% CI for OR* | *χ2 - M-H* | *P* | *χ2 Yates* | *P* |
| *G vs C* | *Resistant vs COPD (total)* | *3.76* | *1.08-14.29* | *5.62* | *0.02* | *4.45* | *0.03* |
| *CG vs CC* | *Resistant vs COPD(total)* | *6.03* | *1.69-23.43* | *10.97* | *0.0009* | *9.23* | *0.002* |
| | | | | | | | |

| | | | | | | | |
|---|---|---|---|---|---|---|---|
| *OR = odd's ratio, 95% CI = 95% confidence interval* *χ2 - M-H = χ2 Mantel-Haenszel, χ2 Yates = χ2 Yates corrected* | | | | | | | |

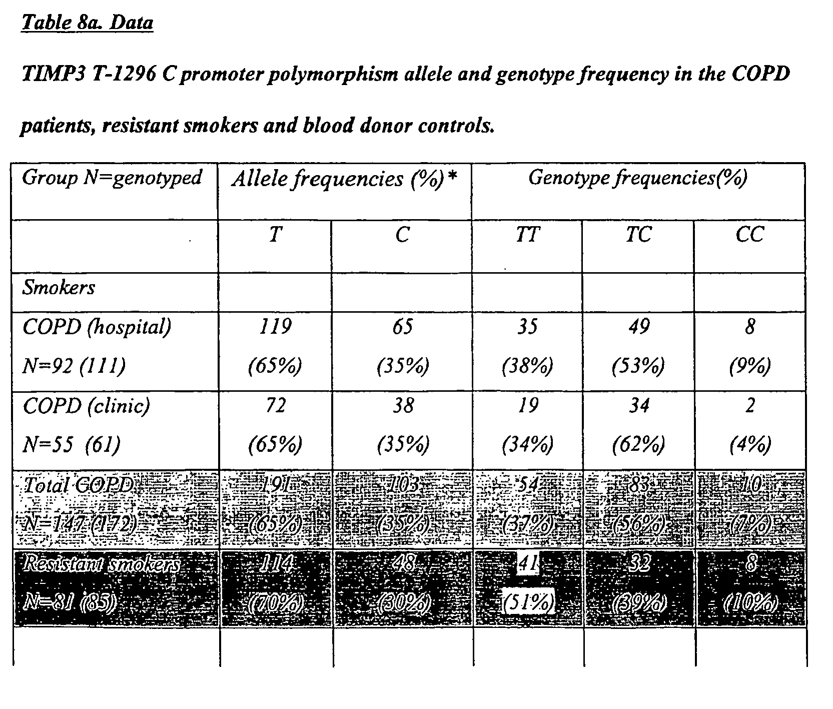

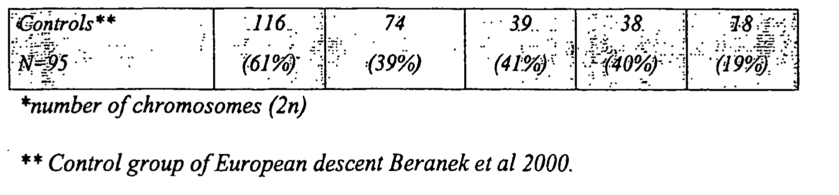

**Table 8b. Analysis**

| ***TIMP3 T-1296 C promoter polymorphism 2x2 contingency table statistics comparing the allele and genotype frequencies in the COPD patients, resistant smokers.*** | | | | | | | |
|---|---|---|---|---|---|---|---|
| *Groups* | *Allele*/*genotye* | *OR* | *95% CI for OR* | *χ2 - M-H* | *P* | *χ2 Yates* | *P* |
| *TT vs TC*/*CC* | *Resistant vs COPD(total)* | *1.77* | *0.98-3.18* | *4.12* | *0.04* | *3.59* | *0.06* |
| *TT vs TC*/*CC* | *Resistant vs COPD(hosp)* | *1.94* | *0.90-4.19* | *3.41* | *0.06* | *2.81* | *0.09* |

| | | | | | | | |
|---|---|---|---|---|---|---|---|
| *OR = odd's ratio, 95% CI = 95% confidence interval* *χ2-M-H = χ2 Mantel-Haenszel, χ2 Yates = χ2 Yates corrected* | | | | | | | |

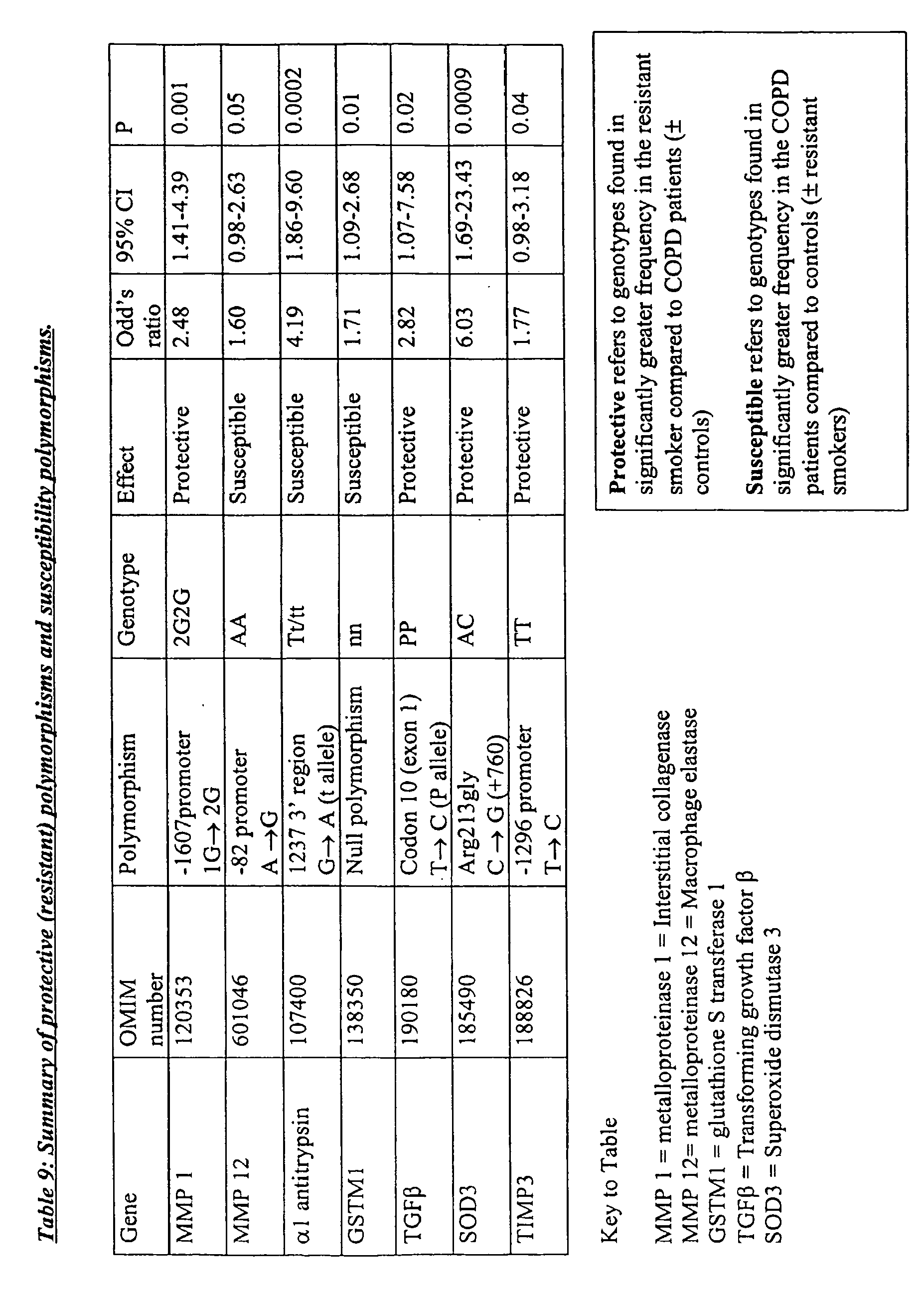

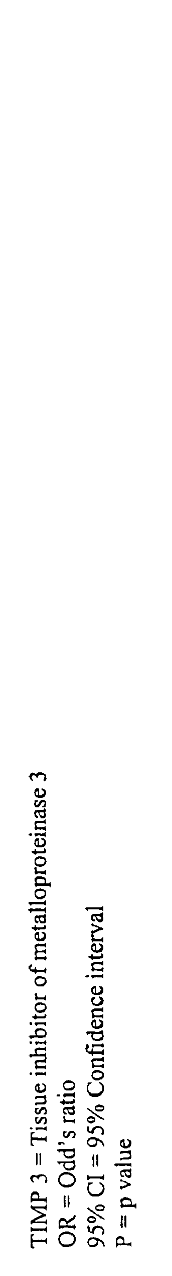

### Summary of the combined analyses

Tables 10 and 11 summarise the results comparing the frequencies of 0, 1 and ≥2 protective or susceptible genotypes between the COPD patients, resistant smokers and controls. Significantly greater number of smokers with COPD had 0 protective genotypes compared to resistant smokers. Conversely, a significantly greater number of resistant smokers had 2 or more protective genotypes compared to smokers with COPD. On comparing the frequencies of 0.1 and ≥2 susceptibility genotypes, significantly greater number of smokers with COPD had ≥2 susceptibility genotypes compared to blood donor controls. A significantly greater number of controls had 0 susceptibility genotypes compared to smokers with COPD.

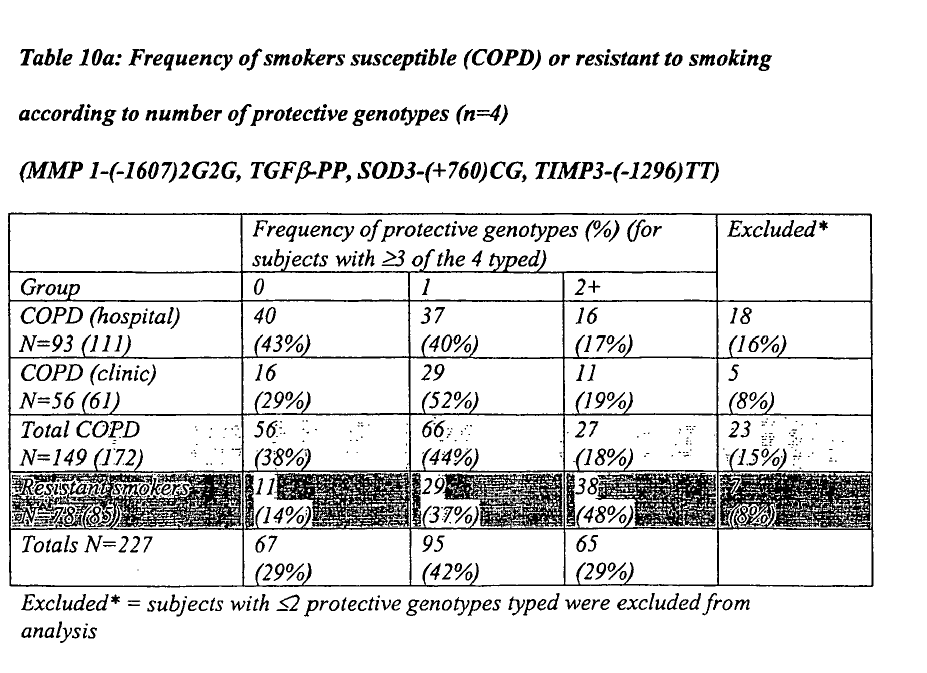

**Table 10b: Frequency of smokers susceptible (COPD) or resistant to smoking according to number of protective genotypes**

| *Genotype* | *Allele*/*genotye* | *OR* | *95% CI for OR* | *χ2 - M-H* | *P* | *χ2 Yates* | *P* |
|---|---|---|---|---|---|---|---|
| *2+vs 0-1* | *Resist vs total COPD* | *4.29* | *2.24-8.27* | *23.35* | *0.000001* | *21.98* | *0.000003* |
| *0 vs1-2+* | *Total COPD vs Resistant* | *3. 67* | *1.70-8.05* | *13.51* | *0.0002* | *12.46* | *0.0004* |
| *2 x 3 table* | *Resist vs total COPD* | - | - | - | - | *26.92* | *0.000001* |

| | | | | | | | |
|---|---|---|---|---|---|---|---|
| *OR = odd's ratio, 95% CI = 95% confidence interval* *χ2- M-H = χ2 Mantel-Haenszel, χ2 Yates = χ2 Yates corrected* | | | | | | | |

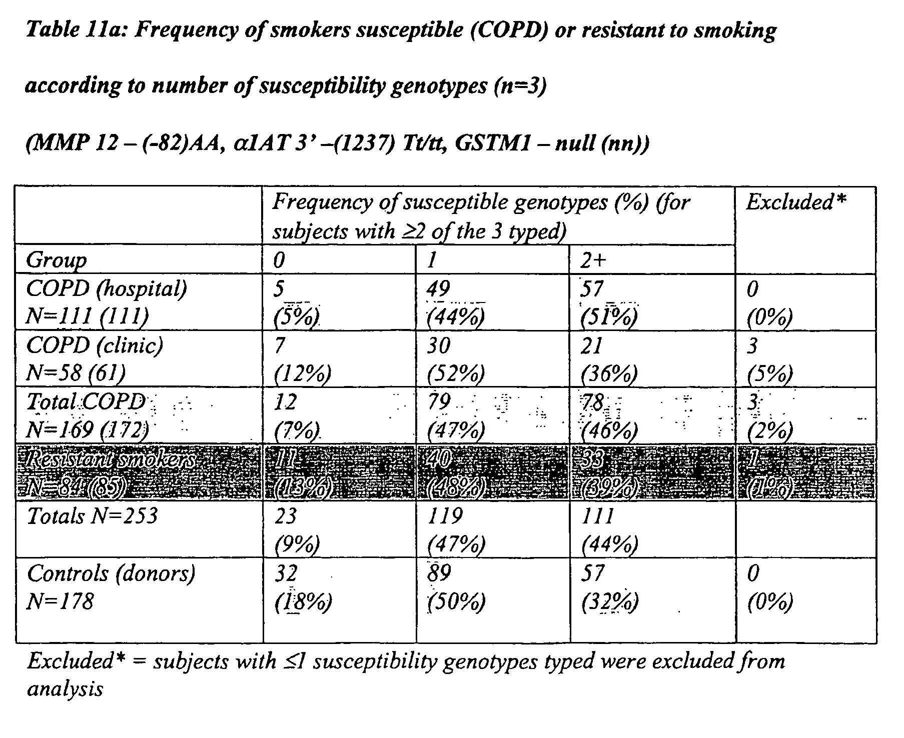

**Table 11b: Frequency of smokers susceptible (COPD) or resistant to smoking according to number of susceptibility genotypes**

| *Genotype* | *Allele*/*genotye* | *OR* | *95% CI for OR* | χ2 *- M-H* | *P* | *χ2 Yates* | *P* |
|---|---|---|---|---|---|---|---|
| *2+ vs 0-1* | *Total COPD vs control* | *1.82* | *1.15-2.88* | *7.26* | *0.007* | *6.70* | *0.009* |
| *2+ vs 0-1* | *COPD (hosp) Vs control* | *2.24* | *1.34-3.76* | *10.66* | *0. 001* | *9.90* | *0.002* |
| *2+ vs 0-1* | *COPD (hosp) vs Resistant* | *1.63* | *0.88-3.01* | *2.78* | *0.10* | *2.34* | *0.13* |
| *2 x* 3 *table* | *Total COPD Vs control* | - | - | - | - | *12.73* | *0.002* |
| *2 x 3 table* | *COPD (hosp) Vs control* | - | - | - | - | *16.66* | *0.0002* |
| *2 x 3 table* | *COPD (hosp) vs Resistant* | - | - | - | - | *5.94* | *0.05* |

| | | | | | | | |
|---|---|---|---|---|---|---|---|
| *OR = odd's ratio, 95% CI = 95% confidence interval* *χ2- M-H = χ2 Mantel-Haenszel, χ2 Yates = χ2 Yates corrected* | | | | | | | |

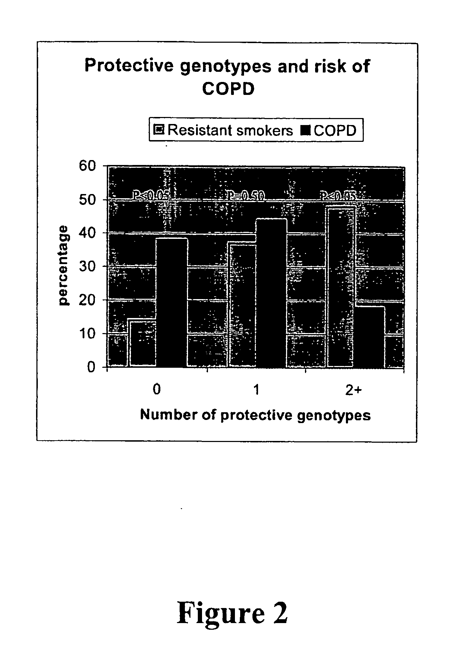

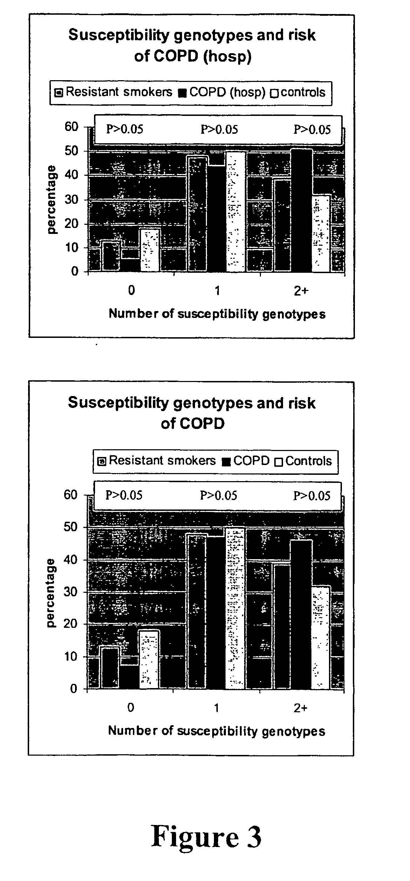

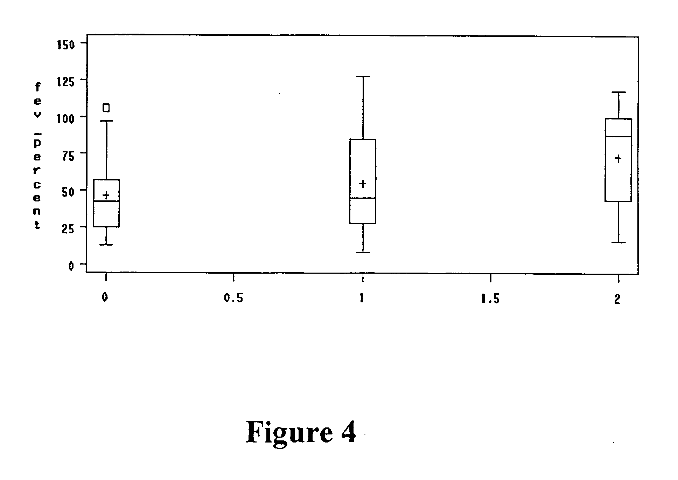

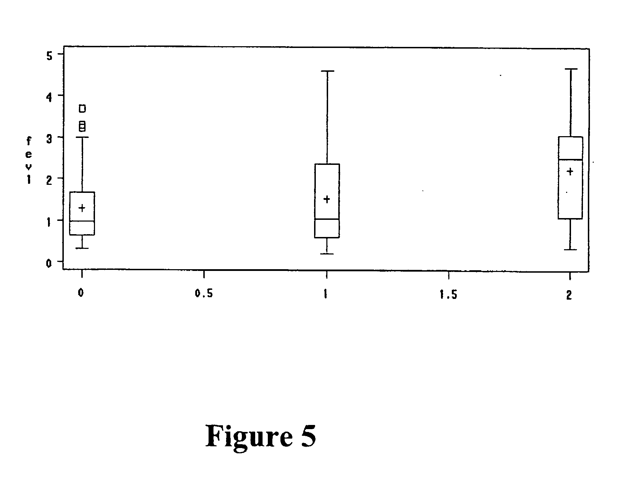

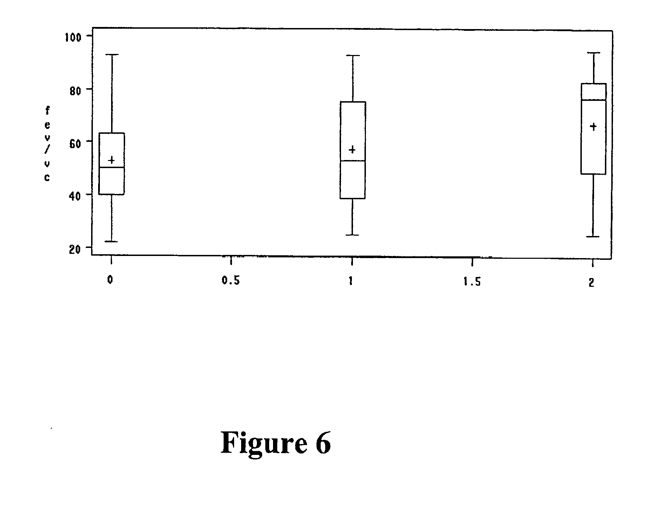

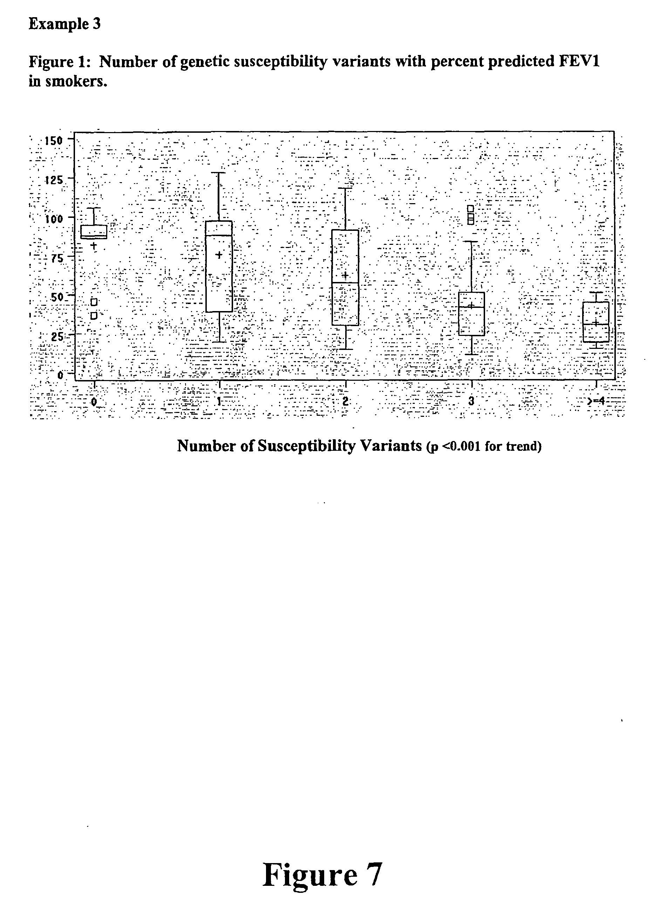

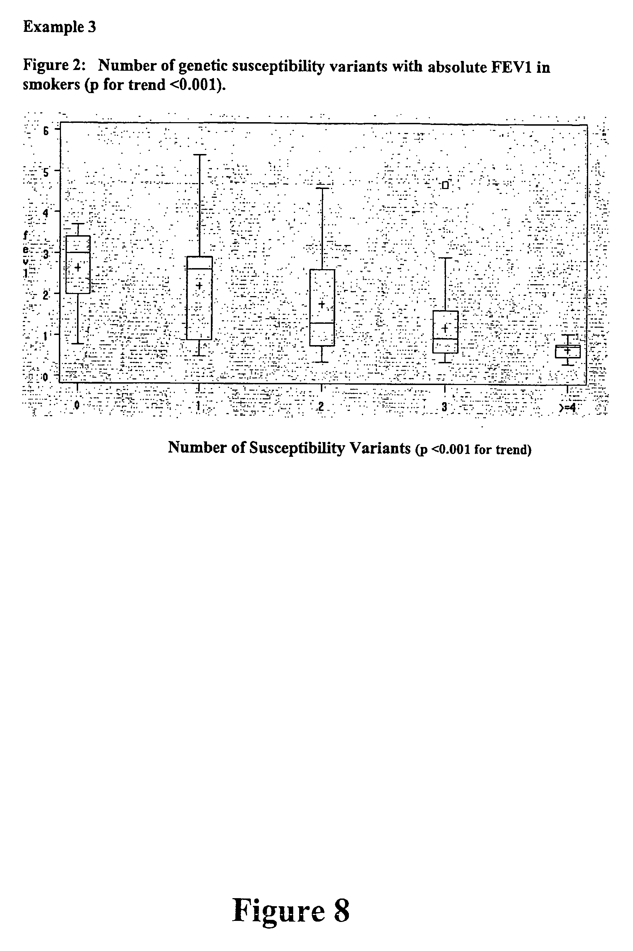

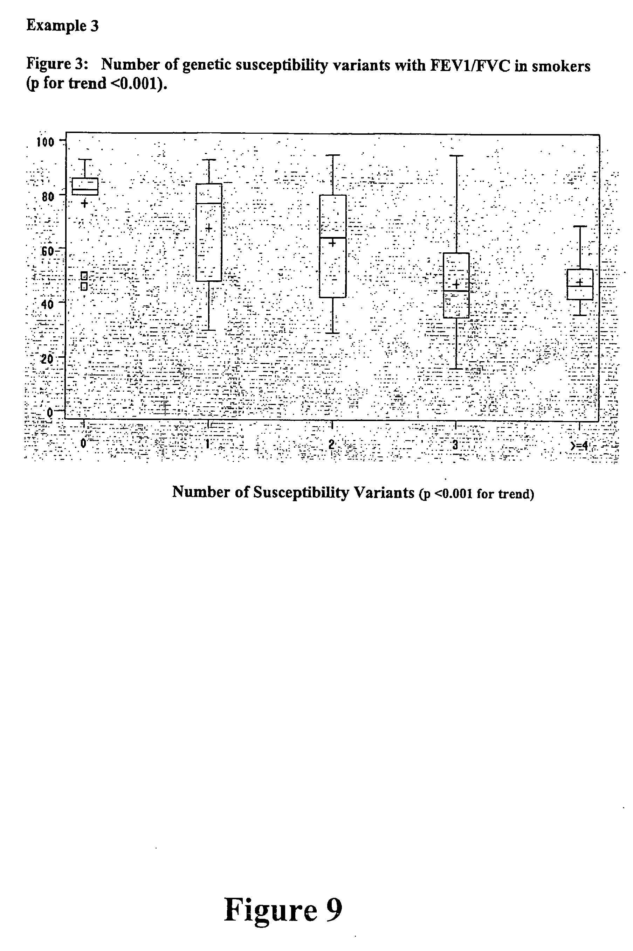

Figure 2 shows graphically the data in Table 10. The risk estimate, as quantified by the standardised ratio, shows that the presence of 0 protective genotypes in this study significantly increases the risk of a smoker having COPD by 167%. Conversely, this analysis shows that the risk of a smoker, with 2 or more protective genotypes, having COPD is reduced by 67%. Given the background risk of COPD in smokers before genetic testing is about 20%, the presence of 0 protective genotypes significantly increases this risk. Figure 3 shows graphically the data in Table 11. Risk estimate analyses showed no significant differences in risk although trends showing a greater risk of a smoker having COPD in the presence of 2 or more susceptibility genotypes was evident. Figures 4-6 show a trend towards greater lung function in smokers with increasing numbers of protective genotypes.

### Discussion of Expanded Study.

*This study shows the novel utility of identifyinggenetic polymorphisms that, alone and in combination, predict increased risk of suffering impaired lung function and COPD from chronic smoking. The basis of this increased risk is likely to be due to (1) the direct effects of those polymorphisms found to alter gene expression or protein function or (2) indirect effects where these polymorphisms are in linkage disequilibrium (genetic variants consistently inherited together) with other mutations that have these direct effects. In keeping with our hypothesis that COPD results from the combined effects of many polymorphisms in smokers, this study has shown that several genes encoding proteins involved in several inter-related pathophysiological processes are involved. Specifically, the results of this study shows that genetic variation in the genes encoding proteins involved in the inflammatory response (TGFβ1), anti-oxidant defence (SOD3, GSTM1) and matrix remodelling (including proteases (MMP1, MMP12) and their inhibitors (α1 antitrypsin, TIMP3)) are likely to contribute to the development of COPD. The genetic variants described here are likely to represent only a sample of the sum total of genetic variants from the above pathophysiological processes that contribute to the development of COPD in smokers but of themselves, significantly increase the ability to identify smokers at higher than average risk for impaired lung function. In this regard, like other biological assays such as serum cholesterol, the methods of the present invention can be used to diagnose a predisposition to future disease before it has become manifest both pathophysiologically or clinically. Epidemiological studies indicate that the processes that result in impaired lung function extend beyond the risk of developing COPD but include coronary artery disease, stroke and lung cancer. Indeed many of the proteins described in this study have been implicated in vascular disease (coronary artery disease and stroke) and cancer suggesting utility in the invention described herein identifying those exposed to cigarette smoke at greater than average risk for vascular disease and cancer.*

### Example 3. Impaired Lung Function Study.

### 3.1. Subject recruitment

Subjects of European decent who had smoked a minimum of twenty pack years were recruited from two sources. The first group were patient who had been diagnosed by a physician to have significantly impaired lung function (in this case labelled as chronic obstructive lung disease) which met the following criteria: were over 50 years old and had developed symptoms of breathlessness after 40 years of age, had a Forced expiratory volume in one second (FEV1) as a percentage of predicted <70% and a FEV1/FVC ratio (Forced expiratory volume in one second/Forced vital capacity) of < 70% (measured using American Thoracic Society criteria). Eighty-four subjects were recruited, of these 56% were male, the mean FEV1/FVC (± Standard Deviation) was 0.44 (0.12), mean FEV 1 as a percentage of predicted was 33 (13). Mean age and pack year history was 73 yrs (9) and 45 pack years (29) respectively. Using a PCR based method (Sandford et al., 1999), we genotyped the COPD group for the α1-antitrypsin mutations (S and Z variants) and none had the Z allele (MZ, SZ, ZZ genotype). We also studied 58 European subjects who had smoked a minimum of twenty pack years and who had never suffered breathlessness and had not been diagnosed with an obstructive lung disease in the past, in particular asthma or chronic obstructive lung disease. This control group was recruited through clubs for the elderly and consisted of 57% male, the mean FEV1/FVC (± Standard Deviation) was 0.82 (0.08), mean FEV1 as a percentage of predicted was 97 (11). Mean age and pack year history was 54 yrs (11) and 46 pack years (28) respectively.

Genotyping methods and candidate genes are as described in earlier examples.

### 3.2 Results comparing the five genetic variants individually and collectively in smokers with normal and impaired lung function.

When the smokers were subdivided in to tertiles of lung function (FEV 1 percent predicted, absolute FEV1 and FEV1/FVC) we find a significant trend towards an excess of the susceptibility genetic variants in the lowest tertile groups (see tables 1-3 below).

**Table 1. Subjects by tertiles of FEV1 percent predicted and frequency of susceptibility variants (in bold).**

| | A-82G HME | | C-1562T Gel B | | 1G/2G MMP 1 | | G1237A 3' α1-AT | | GSTM1 null | |
|---|---|---|---|---|---|---|---|---|---|---|
| Tertile | aa | ag/gg | CC | CT/ TT | 1G1G 1G2G | 2G2G | TT | Tt/tt | N | n |
| Lowest | 37 (79%) | 10 (21%) | 26 (55%) | 21 (45%) | 30 (64%) | 17 (36%) | 37 (79%) | 10 (21%) | 12 (25%) | 35 (74%) |
| Middle | 36 (73%) | 13 (27%) | 29 (59%) | 20 (41%) | 29 (59%) | 20 (41%) | 40 (82%) | 9 (18%) | 23 (48%) | 25 (52%) |
| highest | 17 (37%) | 29 (63%) | 36 (78%) | 10 (21%) | 33 (72%) | 13 (28%) | 44 (96%) | 2 (4%) | 19 (45%) | 23 (55%) |
| P value* | <0.0001 | | 0.05 | | Ns | | 0.05 | | 0.05 | |

| | | | | | | | | | | |
|---|---|---|---|---|---|---|---|---|---|---|
| HME= human macrophage elastase (MMP 12), Gel b= gelatinase B (MMP 9), MMP 1= matrix metalloproteinase 1 or interstitial collagenase, α1-AT= α1-antitrypsin, GSTM 1 = glutathione S transferase M. *= p value for Chi-square. | | | | | | | | | | |

**Table 2. Subjects by tertiles of absolute FEV1 and frequency of susceptibility variants (in bold).**

| | A-82G HME | | C-1562T Gel B | | 1G/2G MMP 1 | | G1237A 3' α1-AT | | GSTM1 null | |
|---|---|---|---|---|---|---|---|---|---|---|
| Tertile | aa | ag/gg | CC | CT/ TT | 1G1G 1G2G | 2G2 G | TT | Tt/tt | N | n |
| Lowest | 36 (78%) | 10 (22%) | 20 (43%) | 26 (57%) | 31 (67%) | 15 (33%) | 36 (78%) | 10 (22%) | 10 (22%) | 36 (78%) |
| Middle | 33 (70%) | 14 (30%) | 32 (68%) | 15 (32%) | 25 (53%) | 22 (47%) | 37 (79%) | 10 (21%) | 21 (45%) | 26 (55%) |
| highest | 21 (43%) | 28 (57%) | 39 (80%) | 10 (20%) | 36 (73%) | 13 (27%) | 48 (98%) | 1 (2%) | 23 (52%) | 21 (48%) |
| P value | 0.0008 | | 0.0009 | | 0.10 | | 0.008 | | 0.008 | |

| | | | | | | | | | | |
|---|---|---|---|---|---|---|---|---|---|---|
| HME= human macrophage elastase (MMP 12), Gel b= gelatinase B (MMP 9), MMP 1= matrix metalloproteinase I or interstitial collagenase, α1-AT= α1-antitrypsin, GSTM1 = glutathione S transferase M. *= p value for Chi-square | | | | | | | | | | |

**Table 3. Subjects by tertiles of FEV1/FVC ratio and frequency of susceptibility variants (in bold).**

| | A-82G HME | | C-1562T Gel B | | 1G/2G MMP 1 | | G1237A 3' α1-AT | | GSTM1 null | |
|---|---|---|---|---|---|---|---|---|---|---|
| Tertile | aa | ag/gg | CC | CT/ TT | 1G1G 1G2G | 2G2 G | TT | Tt/tt | N | n |
| Lowest | 39 (80%) | 10 (20%) | 30 (61%) | 19 (39%) | 29 (59%) | 20 (41%) | 40 (82%) | 9 (18%) | 16 (33%) | 33 (67%) |
| Middle | 34 (74%) | 12 (26%) | 23 (50%) | 23 (50%) | 28 (61%) | 18 (39%) | 37 (80%) | 9 (20%) | 17 (38%) | 28 (62%) |
| highest | 17 (36%) | 30 (64%) | 38 (81%) | 9 (19%) | 35 (74%) | 12 (26%) | 44 (94%) | 3 (6%) | 21 (49%) | 22 (51%) |
| P value | <0.0001 | | 0.007 | | ns | | 0.14 | | 0.11 | |

| | | | | | | | | | | |
|---|---|---|---|---|---|---|---|---|---|---|
| HME= human macrophage elastase (MMP 12), Gel b= gelatinase B (MMP 9), MMP 1= matrix metalloproteinase 1 or interstitial collagenase, α1-AT= α1-antitrypsin, GSTM1 = glutathione S transferase M. *= p value for Chi-square | | | | | | | | | | |

The collective effect of having one or more of the susceptibility variants on lung function is examined in the box plots below. There is a consistent trend towards having progressively worse lung function (FEV1 percent predicted, absolute FEV1 and FEV1/FVC) with increasing number of genetic susceptibility variants (see Figures 1-3)

On logistic regression analysis (after adjusting for all variables) step wise selection found, for each parameter of lung function, several genetic and non-genetic factors that were significantly associated (summarised in table 4 below).

**Table 4. Results of logistic regression analysis for genetic and non-genetic factors associated with lung function.**

| Lung function | Variable | Partial R² | Model R² | P value |
|---|---|---|---|---|
| FEV1 % predicted | age | 0.37 | 0.37 | <0.0001 |
| | A-82G HME | 0.065 | 0.44 | <0.0001 |
| | GSTM1 null | 0.013 | 0.45 | 0.07 |
| Absolute FEV1 | age | 0.52 | 0.52 | <0.0001 |
| | GSTM1 null | 0.026 | 0.54 | 0.006 |
| | A-82G HME | 0.02 | 0.56 | 0.015 |
| | G1237A 3' α1-AT | 0.01 | 0.57 | 0.068 |
| FEV1/FVC | age | 0.34 | 0.34 | <0.0001 |
| | A-82G HME | 0.06 | 0.40 | 0.0003 |
| | Smoking pack years | 0.03 | 0.43 | 0.01 |
| | GSTM1 null | 0.02 | 0.45 | 0.03 |

### Discussion of results.

The results of our study indicate that our susceptibility variants are associated with impaired lung function in a group of smokers. This is the case when we analyse the effect of genotype on lung function as seen in the box plots (figures 1-3). Specifically, the box plot results show that when subjects are divided according to the number of susceptibility variants, a strong inverse linear relationship with impaired lung function is seen, whether the latter is assessed by absolute FEV1, percent predicted FEV1 or FEV1/FVC. Conversely, when subjects are divided according to their lung function in to tertiles, the results consistently show that those with the most impaired lung function have the greatest frequency of our susceptibility genetic variants (see tables 1-3). Lastly, in a stepwise logistic regression, after adjusted for the majority of covariables, the susceptibility variants of the human macrophage elastase gene and glutathione s transferase gene contribute to impaired lung function (see table 4).

With direct relevance to the methods of the present invention and their applications is linkage disequilibrium. This is a phenomenon in genetics whereby two or more mutations or polymorphisms are in such close genetic proximity that they are virtually always co-inherited. This means that in genotyping for one polymorphism, the presence of another polymorphism in linkage disquilibrium can be inferred. Thus any genetic variants (mutations or polymorphisms) that are in linkage disequilibrium with the genetic variants described herein are also included in the inventive concept described herein. Such variants are described in publications included herein as well as in established literature.

The above data provide a novel biological link underlying the epidemiological findings that impaired lung function is not only a useful diagnostic test for predisposition to, and death from, chronic respiratory disease (COPD, asthma, bronchiectasis and bronchiolitis) but also cardiovascular diseases (coronary artery disease and stroke) and certain cancers (lung cancer). The present invention identifies specific genetic susceptibility variants that determine impaired lung function but are also implicated in the underlying pathophysiological processes causing atherogenesis/thrombosis and cancer.

Although the present invention was described with reference to specific examples and preferred embodiments, it will be appreciated by those skilled in the art that variations and modifications which incorporate the principles and the spirit of the inventive concept described herein, are also within the scope of the present invention.

### REFERENCES

Anthonisen NR. 1989 Prognosis in chronic obstructive pulmonary disease: results from a multicenter clinical trial. Am Rev Respir Dis 140; S95-S99.
Bang KM, Gergen PJ, Kramer R, Cohen B. 1993. The effect of pulmonary impairment on all-cause mortality in a National cohort. Chest 103; 536-40.
Barnes PJ. 1999 Molecular genetics of chronic obstructive pulmonary disease. Thorax 54, 245-252.
Beranek M, Kankova K, Muzik J. 2001 Identification of novel common polymorphisms in the promoter region of the TIMP3 gene in Czech population. Mol Cell Probes 14, 265-68.
Calverly PMA. 2000. COPD: early detection and intervention. Chest 117; 365S-371S.
Cantlay AM, Smith CAD, Wallace WA, et al. 1994 Heterogeneous expression and polymorphic genotype of glutathione S-transferases in human lung. Thorax 49, 1010-14.
D'Armiento J, Dalal SS, Okada Y, et al. 1992. Collagenase expression in the lungs of transgenic mice causes pulmonary emphysema. Cell 71, 955-961.
Dalal S, Imai K, Mercer B, et al. 2000 A role for collagenase (Matrix metalloproteinase-I) in pulmonary emphysema. Chest; 117, 227s-228s.
De Boer WI, van Chadewojk A, Sont JK, et al. 1998 Transforming growth factor β1 and recruitment of macrophages and mast cells in airways in chronic obstructive pulmonary disease. Am J Respir Crit Care Med 1998; 158, 1951-195.
Dunleavey L, Beyzade S, Ye S, 2000. Rapid genotype analysis of the metalloproteinase-1 gene 1 G/2G polymorphism that is associated with risk of cancer. Matrix Biol. 19, 175-177.
Fang KC, Wolters PJ, Steinhoff M, et al. 1999 Mast cell expression of Gelatinase A and B is regulated by ket ligand and TGF-beta. 162, 5528-35.
Finlay GA, O'Donnell CM, O'Connor CM, et al. 1996. Elastin and collagen remodeling in emphysema. A scanning electron microscopy study. Am J Pathol 149, 1405-1415.
Finaly GA, O'Driscol LR, Russelll KJ, et al. 1997. Matrix metalloproteinase expression and production by alveolar macrophages in emphysema. Am J Respir Crit Care Med 156, 240-247.
Folz RJ, Guan J, Seldin MF, et al. 1997a Mouse extracellular superoxide dismutase: primary structure, tissue-specific gene expression, chromosomal localisation and lung in situ hybridisation. Am J Resp Cell Mol Biol 17, 393-403.
Folz RJ, Crapo JD, Peno-Green LA. 1997b Elevated levels of extracellular superoxide dismutase in chronic lung disease and characterisation of genetic variants. Chest 111.74S.
Harrison DJ, Cantlay AM, Rae F, et al. 1997 Frequency of glutathione S-transferase M1 deletion in smokers with emphysema and lung cancer. Hum Exp Toxicology 16, 356-60.
Hautamaki R, Kobayashi D, Senior R, Shapiro S. 1997 Requirement for macrophage elastase for cigarette smoke-induced emphysema in mice. Science 277, 2002-2004.
Hole DJ, Watt GCM, Davey-Smith G, et al. 1996. Impaired lung function and mortality risk in men and women: findings from the Renfrew and Paisley prospective population study. BMJ 313; 711-715.
Imai K, Dalal SS, Chen ES, et al. 2001. Human collagenase (matrix metalloproteinase-1) expression in the lungs of patients with emphysema. Am J Respir Crit Care Med 163, 786-91.
Jaumann F. Eissner A, Mazur G, et al. 2000 Transforming growth factor beta 1 is a potent inhibitor of secretory leukocyte protease inhibitor expression in a bronchial epithelial cell line. 15, 1052-7.
Jormsjo S, Ye S, Moritz J, et al. 2000. Allele-specific regulation of matrix metalloproteinase-12 gene activity is associated with coronary artery luminal dimensions in diabetic patients with manifest coronary artery disease. Circ Res 86,998-1003.
Kalsheker NA, Watkins GL, Hill S, et al. 1990 Independent mutations in the flanking sequence of the alphal-antitrypsin gene are associated with chronic obstructive pulmonary disease. Dis Markers 8, 151-57.
Kanamouri Y, Matsushima M, Minaguchi T, et al. 1999. Correlation between expression of the matrix metalloproteinase-1 gene in ovarian cancers and an insertion/delation polymorphism in its promoter region. Cancer Res. 59, 4225-4227.
Knuiman MW, James AL, Divitini M, etal. 1999. Lung function, respiratory symptoms, and mortality: results from the busselton study. Ann Epidemiol 9; 297-306.
Leco KJ, Waterhouse P, Sanchez OH, et al. 2000 Spontaneous air space enlargement in the lungs of mice lacking tissue inhibitor of metalloproteinase -3 (TIMP3) J Clin Invest 108, 817-29.
Loenders B, Van Mechelen E, Nicolai S, et al. 1998 Localisation of extracellular superoxide dismutase in rat lung: neutrophils and macrophages as carriers of the enzyme. 24 1097-1106.
Maniatis,T., Fritsch, E. F. and Sambrook, J., Molecular Cloning Manual. 1989 Marklund SL, Nilsson P, Israelsson K, et al. 1997Two variants of extracellular-superoxide dismutase: relationship to cardiovascular risk factors in an unselected middle-aged population. J Int Med 242, 5-14.
McGowan SE, Jackson SK, Olsen PJ, et al. 1997 Exogenous and endogenous growth factor-beta influence elastin gene expression in cultured fibroblasts. Am J Respir Cell Mol Biol., 17, 25-35.
Ohnishi K, Takagi M, Kurokawa Y, et al.1998 Matrix metalloproteinase-mediated extracellular matrix protein degradation in human pulmonary emphysema. Lab Invest 78 1077-87.
Poller W, Meison C, Olek K. 1990 DNA polymorphisms of the alphal-antitrypsin gene region in patients with chronic obstructive pulmonary disease. Eur J Clin Invest. 20,1-7.
Rahman I, MacNee W. 1999 Lung glutathione and oxidative stress: implications in cigarette smoke induced airway disease. Am J Physiol 277, L1067-88.
Rodriguez BL, Masaki K, Buchfiel C, et al. 1994. Pulmonary function decline and 17-year total mortality: the Honolulu Heart Programme. Am J Epidemiol 140; 398-408.
Rutter JL, Mitchel TI, Buttice G, et al., 1998. A single nucleotide polymorphism in the matrix metalloproteinase-1 promoter creates an Ets binding site and augments transcription. Cancer Res. 58, 5321-5325.
Sandford AJ, Weir TD, Pare PD. 1997. Genetic risk factors for chronic obstructive pulmonary disease. Eur Resp J. 10, 1380-1391.
Sandford AJ, Spinelli JJ, Weir TD, et al. 1997b Mutation in the 3' region of the α1-antitrypsin gene and chronic obstructive pulmonary disease. J Med Genet, 34, 874-5.
Sandford AJ, Weir TD, Spinelli JJ, Pare PD. 1999. Z and S mutations of the α1-antitrypsin gene and the risk of chronic obstructive pulmonary disease. Am J Respir Cell Mol Biol. 20, 287-291.
Segura-Valdez L, Pardo A, Gaxiola M, et al. 2000. Upregulation of Gelatinase A and B, Collagenase 1 and 2, and increased parenchymal cell death in COPD. Chest 117, 684-694.
Shapiro SD. 1999. Diverse roles of macrophage matrix metalloproteinases in tissue destruction and tumour growth. Thromb Haemost. 82, 846-849.
Sorlie PD, Kannel WB, O'Conner G. 1989. Mortality associated with respiratory function and symptoms in advanced age: the Framingham study. Am Rev Respir Dis 140; 379-384.
Subramanian D, Guntupalli KK. 1994. Diagnosing obstructive lung disease. Why is differentiating COPD from asthma important? Postgrad Med 95; 69-85.
Suthanthiran M, Li B, Song JO, et al. 2000 Transforming growth factor beta1 hyperexpression in African-American hypertensives: A novel mediator of hypertension and/or target organ damage. PNAS 97, 3479-84.
Tockman MS, Comstock GW. 1989. Respiratory risk factors and mortality: longitudinal studies in Washington county, Maryland. Am Rev Respir dis 140; S56-S63.
Vignola AM, Chanez P, Chiappara G, et al. 1997 Transforming growth factor β expression in mucosal biopsies in asthma and chronic bronchitis. Am J Respir Crit Care Med ,156,591-599.
Von der Ahe D, Nischan C, Kunz C, et al. 1993. Ets transcription factor binding site is required for positive and TNF alpha-induced negative promoter regulation. Nucleic Acids Res 21, 5636-5643.
Wahl SM, Hunt DA, Wakefield LM, et al. 1987 Transforming growth factor beta induces monocyte chemotaxis and growth factor production. Proc Natl Acad Sci USA 5788-92.
Waltenberg J. 2001. Pathophysiological basis of unstable coronary syndrome. Herz 26. Supp 1; 2-8.
Weiss ST, Segal MR, Sparrow D, Wager C. 1995 Relation of FEV1 and peripheral blood leukocyte count to total mortality: the normative aging study. Am J Epidemiol 142; 493-8.
Yang IA, Zimmerman PV, Tunnicliffe AM, et al. 2001 Disease modifying and suceptibility genes in COPD. Respirology 6 Supp, A2.
Ye S. 2000 polymorphisms in matrix metalloproteinase gene promoters:implication in regulation of gene expression and susceptibility of various diseases. Matrix Biol 7; 623-9.
Zhang B, Ye S, Herrmann S-M, et al. 1999 Functional polymorphism in the regulatory region of Gelatinase B gene in relation to severity of coronary atherosclerosis. Circulation; 99:1788-94.

## Claims

1. Method of determining a subject's predisposition to developing chronic obstructive pulmonary disease, comprising the analysis of polymorphisms in the regulatory and/or promoter regions of the genes encoding matrix metalloproteinase, wherein the presence of a mutation in these regions is indicative of predisposition to developing the disease.

2. Method of determining predisposition to developing chronic obstructive pulmonary disease in a subject who is a smoker or someone exposed to high levels of air pollutants such as environmental tobacco smoke, comprising the analysis of polymorphisms in the regulatory and/or promoter region of a matrix metalloproteinase, wherein the presence of a mutation in the regulatory and/or promoter regions of genes encoding matrix metalloproteinases is indicative of predisposition to developing the disease.

3. Method of diagnosing in a subject the potential onset of chronic obstructive pulmonary disease comprising the analysis of polymorphism in the regulatory and/or promoter regions of a matrix metalloproteinase, wherein the presence of a mutation in the promoter region of a matrix metalloproteinase is indicative of potential onset or severity of the disease.

4. Method of diagnosing in a subject who is a smoker or someone exposed to high levels of air pollutants such as environmental tobacco smoke the potential onset of chronic obstructive pulmonary disease comprising the analysis of polymorphism in the regulatory and/or promoter region of a matrix metalloproteinase, wherein the presence of a mutation in the promoter region of a matrix metalloproteinase is indicative of potential onset or severity of the disease

5. A method according to any one of claims 1 to 4, wherein the metalloproteinase is selected from interstitial collagenase (matrix metalloproteinase-1 or MMP-1), gelatinase B (matrix metalloproteinase-9 or MMP-9) and human macrophage elastase gene (MMP-12).

6. A method according to any one of claims 1 to 5, wherein the polymorphism (mutation) is 1G/2G in the promoter of interstitial collagenase, C-1562T in the promoter of gelatinase B and the A to G -82 polymorphism in the promoter of human macrophage elastase gene and/or polymorphisms in linkage disequilibrium with these polymorphisms.

7. A method according to any one of claims 1 to 6, wherein the determination of regulatory and/or promoter polymorphism is conducted on more than one metalloproteinase gene.

8. Method of determining a subject's predisposition to developing chronic obstructive pulmonary disease, comprising the analysis of a combination of polymorphisms in the regulatory and/or promoter regions of the genes involved in, or associated with, matrix remodelling, wherein the presence of a polymorphism in these regions is indicative of predisposition to developing the disease.

9. A method according to claim 8, wherein the determination is conducted on polymorphisms in interstitial collagenase (matrix metalloproteinase-1 or MMP-1), gelatinase B (matrix metalloproteinase-9 or MMP-9) and human macrophage elastase gene (MMP-12).

10. A method according to claim 8 or claim 9, further comprising the determination of polymorphisms in alphal-antitrypsin and/or glutathione S-transferase genes and/or polymorphisms in linkage disequilibrium with these polymorphisms.

11. Set of nucleotide probes and/or primers for use in the methods of any one of claims 1 to 10.

12. A set of nucleotide probes and/or primers according to claim 11, wherein the nucleotide probes and/or primers are those which span, or are able to be used to span, the polymorphic regions of promoters and regulatory regions of the genes.

13. Method of determining a subject's predisposition to developing impaired lung function comprising the analysis of polymorphisms in the genes encoding proteins involved in matrix remodelling, inflammation and response to oxidative stress, wherein the presence of mutations in one or more of said genes is indicative of predisposition to developing impaired lung function.

14. Method of determining predisposition to developing impaired lung function in a subject who is a smoker or a subject exposed to high levels of air pollutants comprising the analysis of polymorphisms in the genes encoding proteins involved in matrix remodelling, inflammation and response to oxidative stress , wherein the presence of mutations in one or more said genes is indicative of predisposition to developing impaired lung function.

15. Method of diagnosing in a subject the potential onset of impaired lung function comprising the analysis of polymorphisms in the genes encoding proteins involved in matrix remodelling, inflammation and response to oxidative stress, wherein the presence of mutations in one or more said genes is indicative of potential onset of impaired lung function.

16. Method of diagnosing in a subject who is a smoker or a subject exposed to high levels of air pollutants the potential onset of impaired lung function comprising the analysis of polymorphisms in the genes encoding proteins involved in matrix remodelling, inflammation and response to oxidative stress, wherein the presence of mutations in one or more of said genes is indicative of predisposition to developing impaired lung function.

17. A method according to any one of claims 1 to 4, wherein the matrix remodelling or inflammatory protein is selected from interstitial collagenase (matrix metalloproteinase-1 or MMP-1), gelatinase B (matrix metalloproteinase-9 or MMP-9), human macrophage elastase gene (MMP-12) and α1-antitrypsin gene and oxidative stress responsive protein glutathione transferases (GSTs).

18. A method according to any one of claims 1 to 5, wherein the polymorphism (mutation) is 1 G/2G in the promoter of interstitial collagenase, C-1562T in the promoter of gelatinase B, the A to G -82 polymorphism in the promoter of human macrophage elastase gene, the G1237A 3' α1-antitrypsin polymorphism, the GSTM1 null polymorphism and/or polymorphisms/mutations in linkage disequilibrium with these polymorphisms.

19. A method according to any one of claims 1 to 6, wherein the determination of functional polymorphisms is conducted on more than one gene encoding proteins involved in matrix remodelling, inflammation and response to oxidative stress.

20. Method of determining a subject's predisposition to developing impaired lung function comprising the analysis of a combination of polymorphisms of the genes involved in, or associated with matrix remodelling, inflammation and response to oxidative stress matrix remodelling, wherein the presence of a polymorphism in these regions is indicative of predisposition to developing impaired lung function.

21. A method according to claim 8, wherein the determination is conducted on polymorphisms in the matrix remodelling or inflammatory protein selected from interstitial collagenase (matrix metalloproteinase-1 or MMP-1), gelatinase B (matrix metalloproteinase-9 or MMP-9), human macrophage elastase gene (MMP-12) and α1-antitrypsin gene and oxidative stress responsive protein glutathione transferases (GSTs).

22. Set of nucleotide probes and/or primers for use in the methods of any one of claims 1 to 10.

23. Method of determining a subject's predisposition to developing morbidity/mortality risk of a disease associated with impaired lung comprising the analysis of polymorphisms in the genes encoding proteins involved in matrix remodelling, inflammation and response to oxidative stress, wherein the presence of mutations in one or more of said genes is indicative of predisposition to developing morbidity/mortality risk of said disease.

24. A method according to claim 11, wherein the disease is selected from a group consisting of chronic obstructive lung diseases, coronary artery disease, stroke and lung cancer.

25. A method according to claim 11 or claim 12, wherein the matrix remodelling or inflammatory protein is selected from interstitial collagenase (matrix metalloproteinase-1 or MMP-1), gelatinase B (matrix metalloproteinase-9 or MMP-9), human macrophage elastase gene (MMP-12) and α1-antitrypsin gene and oxidative stress responsive protein glutathione transferases (GSTs).

26. A method according to any one of claims 11 to 13, wherein the polymorphism (mutation) is 1G/2G in the promoter of interstitial collagenase, C-1562T in the promoter of gelatinase B, the A to G -82 polymorphism in the promoter of human macrophage elastase gene, the G1237A 3' α1-antitrypsin polymorphism, the GSTM1 null polymorphism and/or polymorphisms/mutations in linkage disequilibrium with these polymorphisms.

27. A method according to any one of claims 11 to 14, wherein the determination of functional polymorphisms is conducted on more than one gene encoding proteins involved in matrix remodelling, inflammation and response to oxidative stress.

28. Method of determining a subject's potential risk of developing chronic obstructive pulmonary disease, comprising the analysis of polymorphisms in the regulatory and/or promoter regions of the genes encoding matrix metalloproteinases, inflammatory and anti-inflammatory cytokines, inhibitors of matrix metalloproteinases and enzymes involved in metabolising oxidants, and/or polymorphisms/mutations in linkage disequilibrium with these polymorphisms, wherein the presence of a mutation in these regions is indicative of a genotype protective against COPD.

29. Method of determining the potential risk of developing chronic obstructive pulmonary disease in a subject who is a smoker or someone exposed to high levels of air pollutants such as environmental tobacco smoke, comprising the analysis of polymorphisms in the regulatory and/or promoter regions of the genes encoding matrix metallproteinases inflammatory and anti-inflammatory cytokines, inhibitors of matrix metalloproteinases and enzymes involved in metabolising oxidants and/or polymorphisms/mutations in linkage disequilibrium with these polymorphisms, wherein the presence of a mutation in these regions is indicative of a genotype protective against COPD.

30. Method according to claim 28 or claim 30, wherein the metalloproteinase is interstitial collagenase.

31. Method according to claim 30, wherein the polymorphism is 1G/2G in the promoter of intersitial collagenase and/or polymorphisms/mutations in linkage disequilibrium with this polymorphism.

32. Method according to claim 28 or claim 29, wherein the inflammatory cytokine is transforming growth factor beta1.

33. Method according to claim 32, wherein the polymorphism is substitution of nucleotide T to C in codon 10 of transforming growth factor beta1 and/or polymorphisms/mutations in linkage disequilibrium with this polymorphism.

34. Method according to claim 28 or claim 29, wherein the inhibitor of metalloproteinases is Tissue Inhibitor of Metalloproteinases 3.

35. Method according to claim 34, wherein the polymorphism is substitution of T to C at position -1296 and/or polymorphisms/mutations in linkage disequilibrium with this polymorphism.

36. Method of determining a subject's potential risk of developing chronic obstructive pulmonary disease, comprising the analysis of polymorphisms in the regulatory and/or promoter regions of the genes encoding matrix metalloproteinases, glutathione transferases, and protease inhibitors and/or polymorphisms/mutations in linkage disequilibrium with these polymorphisms, wherein the presence of a mutation in these regions is indicative of a genotype susceptible to COPD.

37. Method of determining the potential risk of developing chronic obstructive pulmonary disease in a subject who is a smoker or someone exposed to high levels of air pollutants such as environmental tobacco smoke, comprising the analysis of polymorphisms in the regulatory and/or promoter regions of the genes encoding matrix metalloproteinases, glutathione transferases, and protease inhibitors and/or polymorphisms/mutations in linkage disequilibrium with these polymorphisms, wherein the presence of a mutation in these regions is indicative of a genotype susceptible to COPD.

38. Method according to claim 36 or claim 37, wherein the metalloproteinase is macrophage elastase (matrix metalloproteinase 12 or MMP12).

39. Method according to claim 38, wherein the polymorphism is substitution of A to G at position -82 and/or polymorphisms/mutations in linkage disequilibrium with this polymorphism.

40. Method according to claim 36 or claim 37, wherein the glutathione transferase is glutathione S transferase 1.

41. Method according to claim 40, wherein the polymorphism is the M1 null polymorphism and/or polymorphisms/mutations in linkage disequilibrium with this polymorphism.

42. Method according to claim 36 or claim 37, wherein the protease inhibitor is alpha1 antitrypsin.

43. Method according to claim 42, wherein the preferred polymorphism is the 3' Taq 1 polymorphism and/or polymorphisms/mutations in linkage disequilibrium with this polymorphism.
